(19) Europäisches Patentamt
European Patent Office
Office européen des brevets

(11) EP 3 882 264 A1

(12) EUROPEAN PATENT APPLICATION

(43) Date of publication:
**22.09.2021 Bulletin 2021/38**

(51) Int Cl.:
***C07K 14/71*** *(2006.01)* ***A61M 1/34*** *(2006.01)*
***A61P 9/12*** *(2006.01)*

(21) Application number: **20164619.7**

(22) Date of filing: **20.03.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universität zu Köln**
**50923 Köln (DE)**

(72) Inventors:
- **Hagmann, Henning**
  **50939 Köln (DE)**
- **Benzing, Thomas**
  **50859 Köln (DE)**
- **Schermer, Bernhard**
  **50321 Brühl (DE)**
- **Koch, Manuel**
  **50829 Köln (DE)**
- **Matin, Mahsa**
  **50935 Köln (DE)**

(74) Representative: **Lahrtz, Fritz**
**Patentanwälte**
**Isenbruck Bösl Hörschler PartG mbB**
**Seckenheimer Landstr. 4**
**68163 Mannheim (DE)**

# (54) VEGF DIMER MOLECULES AND COLUMNS COMPRISING A VEGF DIMER MOLECULE AS WELL AS USES, PRODUCTION METHODS AND METHODS INVOLVING THE SAME

(57) The present invention relates to a column comprising a vascular endothelial growth factor (VEGF) dimer molecule, a method for preparing such a column, a VEGF dimer molecule, an expression vector and a recombinant host cell encoding for a VEGF dimer, as well as uses and methods related thereto.



Printed by Jouve, 75001 PARIS (FR)

## Description

The present invention relates to a column comprising a vascular endothelial growth factor (VEGF) dimer molecule, a method for preparing such a column, a VEGF dimer molecule, an expression vector and a recombinant host cell encoding for a VEGF dimer, as well as uses and methods related thereto.

## Background of the invention

The vascular endothelial growth factor (VEGF) family is a sub-family of growth factors, which is termed the platelet-derived growth factor family of cystine-knot growth factors. Members of the VEGF family are VEGF-A (often referred to as VEGF), VEGF-B, VEGF-C, VEGF-D and Placental growth factor (PIGF). VEGF family members usually function as ligands interacting with at least one of the cellular VEGF receptor tyrosine kinases VEGFR-1, VEGFR-2 and VEGFR-3. Thereby they may operate as important signaling proteins involved in the formation of blood vessels, such as vasculogenesis (blood vessel formation in the embryo by de novo formation of the embryonic circulatory system) and angiogenesis (the formation of blood vessels from pre-existing vessels). Defects in the level of interaction of VEGF family members and their VEGF receptors may lead to severe diseases, such as preeclampsia in pregnant women.

Preeclampsia is a potentially life-threatening multisystem disease affecting 4-8% of pregnant women after the 20th week of gestation (Sibai, 1998; Redman, 2005; Walker 2000). In detail, preeclampsia is usually defined as new-onset hypertension and damage to another organ system, most commonly the kidney indicated by loss of protein in the urine (proteinuria). The condition may progress to life-threatening generalized endothelial dysfunction and thrombotic microangiopathy manifesting with seizures, stroke or multiorgan failure, also called eclampsia or HELLP syndrome (Hemolysis Elevated Liver enzymes Low Platelets).

The pathophysiologic mechanism of preeclampsia is an imbalance of angiogenic and anti-angiogenic factors in the maternal circulation. According to current knowledge, placental ischemia leads to increased expression and systemic release of sFlt-1, the soluble splice variant of VEGF receptor 1 (VEGFR-1), most likely from trophoblast cells (Maynard et al., 2003). sFlt-1 acts as a potent VEGF and placental growth factor (PIGF) antagonist by scavenging and neutralizing these molecules. As a result, the endothelium is deprived of its constant trophic signal, leading to generalized endothelial dysfunction with hypertension and proteinuria being the first hallmark symptoms of disease and, consequently, severe consequences for mother and consequently also her fetus.

In patients with preeclampsia, increased sFlt-1 and decreased circulating PIGF plasma levels are increasingly evolving as powerful predictive markers of imminent disease and can be detected up to five weeks before the onset of symptoms (Levine et al., 2004).

So far, there are only small feasibility pilot studies in clinical trial phase 2 on extracorporeal apheresis (dextran sulfate, H.E.L.P.) and also plasma separation. Effective therapeutic and prophylactic measures for severe preeclampsia are lacking to date except for pre-mature termination of pregnancy (ACOG, 2019). This is especially problematic in patients with early preeclampsia before the $34^{th}$ week estimated gestational age (EGA), when prolongation of pregnancy may be beneficial to the fetus but at the same time detrimental to the mother resulting in an unresolvable therapeutic dilemma.

Therapeutic approaches are e.g., the systemic administration of recombinant proteins, such as $VEGF^{121}$ or PIGF. This resulted in attenuation of symptoms of preeclampsia in animal models (Makris et al., 2016; Li et al., 2007). However, the administration of VEGF and PIGF is limited by serious dose limiting side effects in the mother in case of VEGF and the short half-life limiting the route of PIGF (Eddy et al., 2018). In addition, the administration of stabilized siRNA reduced placental overexpression of sFlt-1 without affecting Flt-1 mRNA in mouse and baboon preeclampsia models (Turanov et al., 2018).

For further development of therapies, understanding the complex pathophysiology of preeclampsia is essential. According to mouse studies and in-situ hybridization experiments in human placenta, trophoblast-derived sFlt-1 protects the fetus from maternal VEGF, which is released in response to placental hypoperfusion and ischemia (Fan et al., 2014). According to this model overflow of placental sFlt-1 into the maternal circulation represents an unintended excess of a primarily physiologic response. Thus, therapeutic approaches targeting the expression of sFlt-1 require caution since they might compromise the physiologic function of sFlt-1 in the placenta and expose the fetus to increased levels of maternal VEGF (Turanov et al., 2018). Circulating sFlt-1 in the maternal blood stream contributes only little to the overall sFlt-1 burden, which mainly accumulates in the extracellular matrix due to its high positive charge. However, only the smaller circulating proportion of sFlt-1 seems to cause endothelial dysfunction and multisystemic disease. Bearing in mind the protective role of sFlt-1 in the placenta and the detrimental function of circulating sFlt-1 the principle of extracorporeal removal of sFlt-1 is captivating.

Therefore, besides the systemic administration of recombinant proteins, extracorporeal strategies are examined to remove sFlt-1 in human pregnancy to restore angiogenic balance in preeclamptic pregnancies. Extracorporeal apheresis of sFlt-1 primarily targets circulating sFlt-1 and only secondarily affects tissue and placental levels of sFlt-1 after redistribution.

One approach is the extracorporeal adsorption of circulating sFlt-1 by ionexchange chromatography by employing dextran sulfate in apheresis of pregnant women with severe early preeclampsia (Thadhani et al., 2011; Thadhani et al., 2016). In this approach, positively charged sFlt-1 is unspecifically retained by the negatively charged dextran sulfate matrix. However, charge dependent ligands, such as dextran sulfate, are very unspecific and may, therefore, also lead to the unwanted removal of other proteins.

Further approaches of extracorporeal adsorption of circulating sFlt-1 are antibody based approaches to reduce sFlt-1 (e.g. US 2010/0247650 A1). However, complexed VEGF and PIGF are also eliminated concomitantly and reduce efficacy of the intervention with regard to restoring the angiogenic balance. Furthermore, the immunogenic potential of sFlt-1 specific antibodies as well as adverse effects due to leakage of antibody from the column are uncertain.

In another approach, ligands, such as PIGF, are immobilized on a matrix and applied in apheresis of pregnant women suffering from preeclampsia (e.g. US 2010/0247650 A1). Thereby, PIGF columns remove sFlt-1 by binding to its receptor site. However, due to the low affinity of PIGF, the capturing PIGF-molecule is not able to compete with complexed VEGF. Therefore PIGF-columns only remove free sFlt-1 or sFlt-1 from circulating PIGF-sFlt-1 complexes. PIGF columns do not remove VEGF-complexed sFlt-1 and do not liberate VEGF. Therefore, this approach only partly removes sFlt-1 and, in comparison to potential competitive apheresis systems, does not restore the angiogenic balance.

A further approach applies magnetic beads functionalized with monomeric truncated VEGF in apheresis to capture sFlt-1 (Trapiella-Alfonso et al., 2019). This approach used a bacterial expressed VEGF truncation ($VEGF^{95}$) and biotinylated beads. However, this approach is not feasible for application in humans due to non-covalent linkage of the ligand to the matrix and unacceptable immunogenity of the avidin-biotin-based interaction (Jain and Cheng, 2017). In addition, the application of bacterial expressed $VEGF^{95}$ has additional limitations. Although glycosylation of VEGF was reported to not affect VEGF binding affinity and activity (Claffey et al., 1995; Peretz et al., 1992), post translational modification specific to bacteria may aggravate immunogenicity (Kuriakose et al., 2016). In addition, the truncated VEGF isoform $VEGF^{95}$ only displays reduced interaction with sFlt-1 due to lacking residues in the receptor binding interface. Also this approach does not lead to the removal of $VEGF^{165}$-complexed sFlt-1 and does not cause the liberation of $VEGF^{165}$.

There is a need to provide means for the treatment of preeclampsia. Especially, there is a need for providing means that allow the efficient removal of sFlt-1 from the blood of patients so that preeclampsia can be efficiently treated in humans.

## Summary of the invention

A column comprising a vascular endothelial growth factor (VEGF) dimer molecule comprising a first and a second VEGF molecule is provided.

The invention further relates to a VEGF dimer molecule comprising a first and a second VEGF molecule, wherein the first and/or the second VEGF molecule have a length of 122 to 250 amino acids.

Moreover, the invention relates to a method for preparing a column of the invention comprising the steps of:

a) preparing by eukaryotic expression a vascular endothelial growth factor (VEGF) dimer molecule, comprising a first and a second VEGF molecule; and
b) immobilizing the dimer of step a) on a matrix.

Further, a VEGF dimer molecule of the invention for use in the treatment of preeclampsia characterized in that the VEGF dimer molecule is bound to a column for apheresis is provided.

The invention further relates to an expression vector comprising a nucleic acid sequence encoding the VEGF dimer molecule of the invention.

Moreover, the invention relates to a recombinant host cell line comprising the VEGF dimer molecule of the present invention, comprising the expression vector of the present invention and/or comprising a nucleic acid sequence encoding the VEGF dimer molecule of the present invention.

A method for separating sFlt-1 from blood and/or releasing VEGF from complexes with sFlt-1 into blood comprising incubating the column or the VEGF dimer molecule of the invention with the blood and separating sFlt-1 from the blood and/or releasing VEGF from complexes with sFlt-1 into the blood is also provided.

In addition, the invention relates to the use of the column or the VEGF dimer molecule of the invention for separating sFlt-1 from blood and/or releasing VEGF from complexes with sFlt-1 into blood.

## Detailed description of the invention

In a first aspect, the invention relates to a column comprising a vascular endothelial growth factor (VEGF) dimer molecule comprising a first and a second VEGF molecule.

Surprisingly, the results of the examples show that sFlt-1 adsorption from human serum was significantly higher for a column comprising a dimer of truncated VEGF of the present invention called $scVEGF^{165}$ in comparison to monomeric PlGF (moPlGF) or monomeric ($moVEGF^{165}$) when coupled to all resins tested. Especially, according to the example, the largest reduction of 86.16 % sFlt-1 in a single run was achieved with columns, where said dimer of the invention ($scVEGF^{165}$) was immobilized on an agarose matrix (Fig. 3A). The examination of longitudinal stability of $scVEGF^{165}$ agarose column further revealed only a slight decrease in binding sFlt-1 (Fig. 3B). The release of PlGF and, therefore, the increase of the PlGF concentration in the blood was most pronounced for the dimer of the invention, while apheresis with $moVEGF^{165}$ only resulted in a release of PlGF half that much of $scVEGF^{165}$. Strikingly, release of sFlt-1 bound VEGF was most pronounced for $scVEGF^{165}$ (Fig. 4), which implies competitive binding of $scVEGF^{165}$ to sFlt-1 due to higher binding affinity. Accordingly, it could be shown that the binding affinity of $scVEGF^{165}$ to VEGF-trap unexpectedly is 11% higher in comparison to that of $moVEGF^{165}$, especially in light of the fact that the binding affinity of scPlGF in comparison to moPlGF is about equal (Fig. 2).

In general, it can be concluded from the results of the examples shown below that the unique characteristic of an apheresis with a VEGF dimer molecule of the invention compared to antibody-mediated apheresis and monomeric VEGF- or PlGF-based approaches is the enhanced affinity for sFlt-1 and thus the capability to efficiently release VEGF and PlGF (Fig. 6).

To sum up, the invention describes for the first time a specific VEGF-based sFlt-1 apheresis system to treat preeclampsia that is applicable in vivo. By exploiting the unique molecular characteristics of the VEGF molecule, it was possible to generate highly specific apheresis columns to immobilize sFlt-1 and at the same time competitive release complexed VEGF and PlGF to restore the angiogenic balance in patients with preeclampsia. Due to the complex and highly structured morphology of VEGF dimers exposing the receptor binding site, use of full-length VEGF dimers as compared to shorter peptide sequences yield better affinity.

Columns for performing apheresis are known in the art (Thadhani et al., 2011; Thadhani et al., 2016). The term "column" according to the present invention describes any device comprising a lumen with two open ends. For example, this means that in case of a column for apheresis, blood from the patient is led into the lumen of the column via the first open end, and, after passing the lumen, is led out of the lumen via the second open end.

The column of the present invention usually is capable of carrying the matrix for a chromatographic separation method. Preferably, the column of the invention is suitable for separating components of a mixture, such as blood or blood plasma. For example, the column may be a chromatography column, such as used for affinity chromatography.

The column may further be made of any material known to be suitable for a chromatography column by the person skilled in the art, such as metal, glass and/or plastic (e.g. polycarbonate). In general, the material is suitable for medical applications, such as apheresis. Moreover, the column may have any size or volume. Preferably, the column has a volume suitable to be applicable in apheresis (e.g. from 20 to 300 ml). Moreover, the column of the invention may have any shape known to the person skilled in the art to be suitable for chromatography methods, such as a cylindrical shape or a tube. Usually, the cavity of the column according to the present invention is packed with the matrix.

The column may be suitable for being applied in medical methods, such as in extracorporeal therapy, e.g. apheresis, whereby the blood or blood plasma of a person may be passed through the column leading to the separation of one or more particular constituent and returning the remaining one or more constituents to the circulation. Moreover, the column may be sterilized by e.g. irradiation, such as gamma-ray irradiation, electron beam irradiation or x-ray irradiation.

The column of the present invention comprises a vascular endothelial growth factor (VEGF) dimer molecule comprising a first and a second VEGF molecule.

VEGF molecules are known in the art. The VEGF molecule comprised according to the present invention in the VEGF dimer molecule may be any vascular endothelial growth factor protein molecule known to the person skilled in the art including truncated forms thereof and may be from any species. For example, the VEGF molecule may be mammalian VEGF, such as mouse, rat or human VEGF. Preferably, the VEGF molecule is human VEGF. Preferably the VEGF molecule is VEGF-A, VEGF-B, VEGF-C, VEGF-D or PlGF, more preferably the VEGF molecule is VEGF-A, VEGF-B or PlGF, even more preferably the VEGF molecule is VEGF-A or VEGF-B and most preferably the VEGF molecule is VEGF-A.

Preferably the VEGF molecule is human VEGF-A, human VEGF-B, human VEGF-C, human VEGF-D or human PlGF, more preferably the VEGF molecule is human VEGF-A, human VEGF-B or human PlGF, even more preferably the VEGF molecule is human VEGF-A or human VEGF-B and most preferably the VEGF molecule is human VEGF-A.

The VEGF molecule comprised in the VEGF dimer of the column of the first aspect of the invention may be of any length. The VEGF molecule comprised in the VEGF dimer of the column of the first aspect of the invention may be full-length VEGF, preferably full-length VEGF-A. For example, the VEGF molecule may comprise or consist of the sequence of human full-length VEGF-A (SEQ ID NO: 1).

Moreover, the VEGF molecule may be a truncated form of VEGF, such as a truncated form of VEGF-A. Preferably the VEGF molecule of the column of the first aspect of the invention may be a truncated form of human VEGF, more preferably a truncated form of human VEGF-A, such as human VEGF-$A^{121}$, human VEGF-$A^{138}$, human VEGF-$A^{145}$, human VEGF-$A^{162}$, human VEGF-$A^{165}$, human VEGF-$A^{165b}$, human VEGF-$A^{189}$ or human VEGF-$A^{206}$ (whereby the numbers correspond to the numbers of proteins in the respective protein based on the natural human VEGF-A sequence).

In an especially preferred embodiment, the VEGF molecule is human VEGF-$A^{165}$.

The truncated VEGF molecule may only comprise a truncated natural VEGF molecule sequence or additional extensions as describe below. For example, the first and/or the second VEGF molecule may be truncated down to a length of 122 amino acids, preferably 165 amino acids.

Preferably, the first VEGF molecule comprised in the VEGF dimer of the column of the first aspect of the invention comprises the N-terminal helix (24-35), the loop connecting β3 to β4 (69-74) and strand β7 (111-114) and the second VEGF molecule comprises residues from strand β2 (54-56) and from strands β5 and β6 together with the connecting turn (79- 91). In that case, the first and second VEGF molecule may dimerize in anti-parallel orientation and, thereby, together comprise the region responsible for sFlt-1 binding when forming the VEGF dimer molecule, i.e. sFlt-1 the binding site may be composed of respective residues from both monomers.

It is also possible in case of a VEGF dimer molecule as used in the column of the first aspect of the invention that the truncated first VEGF molecule consists of amino acids 1 to 114, i.e. comprising the sFlt-1 binding site comprising the N-terminal helix (24-35), the loop connecting β3 to β4 (69-74) and strand β7 (111-114), and the truncated second VEGF monomer consists of amino acids 1 to 91, comprising the residues from strand β2 (54-56) and from strands β5 and β6 together with the connecting turn (79- 91).

Also preferably, the first and/or second VEGF molecule comprise the VEGF amino acid residues Asp63, Glu64 and Glu67 (numbers correspond to those of the natural occurring human VEGF-A amino acid sequence).

Moreover, the amino acid sequence of the VEGF molecule may be a natural occurring sequence or may be mutated. The VEGF molecule may comprise no, one, two, three, four or more mutations. Mutations may affect any amino acid of a VEGF molecule. For example, amino acids may be mutated to enhance binding affinity of a VEGF molecule to sFlt-1. Mutations may also comprise the exchange of one or more amino acids for amino acids having the same or similar charge and/or size.

The VEGF molecule may comprise any form of posttranslational modification known to the person skilled in the art, such as phosphorylation, glycosylation and/or acetylation. Moreover, the VEGF molecule may comprise one, two, three, four or more intramolecular disulfide bonds.

Further, in the column of the present invention, the first and the second VEGF molecule are part of a vascular endothelial growth factor (VEGF) dimer molecule.

The term "VEGF dimer molecule" according to the present invention describes any molecule comprising at least two VEGF molecules, preferably two VEGF molecules.The at least two VEGF molecules that are part of the VEGF dimer molecule may interact via one, two, three, four or more amino acids by ionic binding, covalent binding, van der Waals forces, hydrogen bridge bonds, hydrophobic interactions and/or electrostatic interaction.

Preferably, the at least two VEGF molecules that are part of the VEGF dimer molecule may be connected by at least one covalent bond. More, preferably the at least two VEGF molecules that are part of the VEGF dimer molecule may be connected by one covalent bond, e.g. forming a single-chain molecule. For example, in case of a first and a second VEGF molecule, the nucleic acid sequences encoding of both VEGF molecules may be connected to one nucleic acid sequence molecule leading to the expression of one amino acid molecule comprising both VEGF molecules.

The first and the second VEGF molecule in a VEGF dimer molecule may further be linked by one, two, three, four or more intermolecular and/or intramolecular disulfide bonds. For example, the VEGF dimer molecule may form an open structure of two VEGF molecules (such as $scVEGF^{165}$ molecules) linked by one or more intramolecular disulfide bonds.

Alternatively, the first and the second VEGF molecule in a VEGF molecule dimer may not be linked by any intramolecular disulfide bond.

Any amino acid involved in one of above-mentioned intramolecular or intermolecular disulfide bridges or being directly or sterically adjacent may be mutated.

Dimerization of the first and the second VEGF molecule to form a VEGF dimer molecule may occur in an antiparallel side-by-side fashion with intermolecular disulfide bonds between the single monomers generating a planar surface for the interaction with the receptor protein (Flt-1/sFlt-1). The Flt-1 binding site may comprise residues of 3 beta sheets (β3; β4; β7) as well as the N-terminal helix of one monomer and residues from 3 complementary beta sheets (β2; β5; β6) from the other. Mutational strategies to enhance VEGF-affinity for sFlt-1 may affect any one of these residues or none of them at all.

Moreover, also steric effects may affect sFlt-1 affinity in multimeric forms of VEGF and PIGF and, therefore, may also be altered by one or more mutations.

Moreover, the VEGF dimer molecule may further be part of a VEGF multimer molecule, such as a VEGF trimer, a VEGF tetramer or any form of a VEGF multimer comprising any number of VEGF molecules. Therefore, the column may also comprise a VEGF trimer, a VEGF tetramer or any form of a VEGF multimer. Further, the column may comprise VEGF molecules of one multimer type only, such as only VEGF dimers, only VEGF trimers or only VEGF tetramers, or the column may comprise any mixture of multimers, such as a mixture of VEGF dimers, VEGF trimers, VEGF tetramers and/or VEGF multimers comprising any number of VEGF molecules.

Moreover, the VEGF dimer molecule may be part of a VEGF multimeric structure. For example, a first VEGF dimer molecule may interact (e.g. via intermolecular disulfide bonds) with a second VEGF dimer molecule allowing the formation of tetramers, or multimeric chains of VEGF molecules.

In an especially preferred embodiment, the VEGF dimer molecule of the column of the invention is the VEGF dimer molecule according to the second aspect of the invention discussed below.

In a second aspect, the invention relates to a VEGF dimer molecule comprising a first and a second VEGF molecule, wherein the first and/or the second VEGF molecule have a length of 122 to 250 amino acids.

Except for the length of the first and/or second VEGF molecule, which in the second aspect of the invention is restricted to 122 to 250 amino acids, while there are no restrictions in VEGF molecule according to the first aspect of the invention, all embodiments and features described above with respect to the first and second VEGF molecule of the column according to the first aspect of the invention also apply to this second aspect. For example, if the first and/or second VEGF molecule are truncated, the VEGF molecule of the second aspect of the invention preferably may be a truncated form of human VEGF having a length of 122 to 250 amino acids, more preferably a truncated form of human VEGF-A having a length of 122 to 250 amino acids, such as, human VEGF-$A^{138}$, human VEGF-$A^{145}$, human VEGF-$A^{162}$, human VEGF-$A^{165}$, human VEGF-$A^{165b}$, human VEGF-$A^{189}$ or human VEGF-$A^{206}$ (whereby the numbers correspond to the numbers of proteins in the respective protein based on the natural human VEGF-A sequence).

Further, with the exception of the restriction to the lengths of the first and/or second VEGF molecule, all embodiments and features described above with respect to the VEGF dimer molecule of the column according to the first aspect of the invention also apply to the second aspect.

The following embodiments further describe both, the column of the first aspect of the invention and the VEGF dimer molecule of the second aspect of the invention, if not indicated that they only concern the first or second aspect only.

The VEGF dimer molecule of the column according to the first aspect of the invention or the VEGF dimer molecule according to the second aspect of the invention can be chemically synthesized by conventional methods. Methods for chemically synthesizing the VEGF dimer molecule of the column according to the first aspect of the invention or the VEGF dimer molecule according to the second aspect of the invention are well known to the person skilled in the art.

Furthermore, the VEGF dimer molecule may be produced by recombinant protein expression, e.g. it may be expressed in eukaryotic cells, such as in human cells lines (e.g. HEK293T Epstein-Barr virus nuclear antigen (EBNA) cell lines), mouse cell lines, rat cell lines, hamster cell lines, yeast cells or insect cells. Suitable methods for recombinant protein expression of the VEGF dimer molecule are well known to the person skilled in the art. For example, uncloned, double-stranded linear DNA fragments containing a nucleotide sequence representing the VEGF dimer may be customized, e.g. by codon optimization, and produced. Usually, DNA fragments are amplified using primers and cloned into an expression vector (such as the sleeping beauty transposon expression vector) carrying a protein tag, such as an N-terminal strep tag. In general, stable cell lines are generated by transfecting the vectors into the cells using a transfection method, such as applying a transfection reagent or electroporation. Afterwards, the protein may be isolated from the cells by harvesting the supernatant or the whole culture (if the protein is not secreted by the cells).

Subsequently, the produced VEGF dimer molecule is usually isolated and purified. Methods for isolating and purifying the VEGF dimer molecule of the column according to the first aspect of the invention or the VEGF dimer molecule according to the second aspect of the invention are well known to the person skilled in the art. For example, the VEGF dimer molecule can be purified using a filter technology or chromatography technique, such as affinity chromatography, high performance liquid chromatography (HPLC) or ion exchange chromatography, gel filtration, or other known methods. For example, the VEGF dimer molecule may be linked to and expressed with a protein-tag, such as a Strep tag or a glutathione S-transferase tag (GST-tag), Histidine-tag (HIS-tag) which allows purification via an affinity chromatography column, such as StrepTactin (e.g. Strep-Tactin®XT (IBA Lifesci-ence, Göttingen, Germany)) or glutathione or nickle affinity chromatography. During affinity chromatography or afterwards, the protein-tag is usually cut off from the VEGF dimer molecule by a protease. For example, the Strep tag may be cut off from the VEGF dimer molecule using thrombin protease.

The VEGF dimer molecule of the column according to the first aspect of the invention or the VEGF dimer molecule according to the second aspect of the invention may be synthesized or expressed as one molecule. Alternatively, The VEGF dimer molecule of the column according to the first aspect of the invention or the VEGF dimer molecule according to the second aspect of the invention may be synthesized or expressed in at least two parts and subsequently be connected. For example, the first and the second VEGF molecule of the VEGF dimer of the column of the first aspect of the invention or of the VEGF dimer of the second aspect of the invention may be synthesized or expressed separately and subsequently be connected.

Moreover, the VEGF dimer molecule or the first and/or the second VEGF molecule may be chemically modified, e.g. by adding chemical groups, such as posttranslational modifications. Suitable chemical modifications as well as methods for chemical modification are well known to the person skilled in the art, such as phosphorylation, glycosylation and/or acetylation.

In a preferred embodiment, the VEGF dimer molecule of the column according to the first aspect of the invention or the VEGF dimer molecule according to the second aspect of the invention is expressed by a eukaryotic cell.

The first and the second VEGF molecule of the column according to the first aspect of the invention or of the VEGF dimer molecule according to the second aspect of the invention may be different. For example, they may differ in their length, in their amino acid sequence and/or in their posttranslational modifications. Alternatively, the first and the second VEGF molecule of the column according to the first aspect of the invention or of the VEGF dimer molecule according to the second aspect of the invention may be identical.

In a further preferred embodiment, the first and the second VEGF molecule of the column according to the first aspect of the invention or of the VEGF dimer molecule according to the second aspect of the invention are identical.

The first and/or the second VEGF molecule of the column according to the first aspect of the invention or of the VEGF dimer molecule according to the second aspect of the invention may lack a part of or the whole VEGF N-terminal signal peptide MNFLLSWVHWSLALLLYLHHAKWSQA (SEQ ID NO: 2).

The part or the whole VEGF N-terminal signal sequence may be cleaved from the VEGF dimer sequence or the first and/or second VEGF molecule sequence before they are introduced in an expression vector or in the expression vector before introducing it into cells for recombinant expression. Moreover, the part or the whole VEGF N-terminal signal peptide may be cleaved from the synthesized or expressed first and/or second VEGF molecule before connecting them to a dimer or may be cleaved from the synthesized or expressed second VEGF molecule after connecting it to the first VEGF molecule to form a dimer. Methods for cutting of the N-terminal signal sequence from the VEGF nucleic acid or amino acid sequence are well known to the person skilled in the art as well as suitable nucleases or proteases.

In another preferred embodiment, the first and/or the second VEGF molecule of the column according to the first aspect of the invention or of the VEGF dimer molecule according to the second aspect of the invention lack the N-terminal signal peptide.

Moreover, the first and/or the second VEGF molecule of the column according to the first aspect of the invention or of the VEGF dimer molecule according to the second aspect of the invention may be a full-length or truncated VEGF molecule extended by additional amino acids. In detail, the first and/or second VEGF molecule may be extended up to a length of 250 amino acids on their N- and/or C-terminus. Preferably, the first VEGF molecule is extended on its N-terminus and/or the second VEGF monomer is extended on its C-terminus. This may have the advantage that a possible misfolding of the first and the second VEGF molecule is prevented. For example, the first and/or the second VEGF molecule may be extended by a protein tag, such as a GST-tag, a HIS-tag, a Strep-tag or by addition of any sequence of amino acid residues. Moreover, the first and/or second VEGF molecule may still comprise remnants originally required for their production, such as restriction sites.

The first and/or the second VEGF molecule of the column according to the first aspect of the invention or of the VEGF dimer molecule according to the second aspect of the invention may have a length of 122 to 250 amino acids, preferably 125 to 225 amino acids, more preferably 140 to 200 amino acids, even more preferably 150 to 180 amino acids, especially more preferably 160 to 170 amino acids and most preferably 165 amino acids..

In a preferred embodiment, the first and/or the second VEGF molecule of the column according to the first aspect of the invention or of the VEGF dimer molecule according to the second aspect of the invention have a length of 122 to 250 amino acids, preferably 125 to 225 amino acids, more preferably 140 to 200 amino acids, even more preferably 150 to 180 amino acids, especially more preferably 160 to 170 amino acids and most preferably 165 amino acids.

Moreover, the first VEGF molecule of the column according to the first aspect of the invention or of the VEGF dimer molecule according to the second aspect of the invention may have at least 50%, at least 55%, at least 60%, at least 65%, at least 70%, at least 75%, at least 80%, at least 85%, at least 90%, at least 95%, or 100% (e.g., at least 86%, at least 87%, at least 88%, at least 89%, at least 90%, at least 91%, at least 92%, at least 93%, at least 94%, at least 95%, at least 96%, at least 97%, at least 98% or at least 99% amino acid sequence identity in comparison to SEQ ID NO: 3 and/or the second VEGF molecule of the column according to the first aspect of the invention or of the VEGF dimer molecule according to the second aspect of the invention may have at least 50%, at least 55%, at least 60%, at least 65%, at least 70%, at least 75%, at least 80%, at least 85%, at least 90%, at least 95%, or 100% (e.g., at least 86%, at least 87%, at least 88%, at least 89%, at least 90%, at least 91%, at least 92%, at least 93%, at least 94%, at least 95%, at least 96%, at least 97%, at least 98% or at least 99% amino acid sequence identity in comparison to SEQ ID NO: 4.

The term "at least 50% amino acid sequence identity" as used herein means that the amino acid sequence of the first and/or second VEGF molecule of the column according to the first aspect of the invention or of the VEGF dimer molecule according to the second aspect of the invention has an amino acid sequence characterized in that, within a stretch of 100 amino acids, at least 50 amino acid residues are identical to the sequence of the corresponding sequence, e.g. of SEQ ID No: 3 and/or SEQ ID No: 4, respectively.

Sequence identity according to the present disclosure invention can, e.g., be determined by methods of sequence alignment in form of sequence comparison. Methods of sequence alignment are well known in the art and include various programs and alignment algorithms. Moreover, the NCBI Basic Local Alignment Search Tool (BLAST) is available from several sources, including the National Center for Biotechnology Information (NCBI, Bethesda, MD) and on the internet, for use in connection with the sequence analysis programs blastp, blastn, blastx, tblastn and tblastx. Percentage of identity of mutants according to the present disclosure invention relative to the amino acid sequence of e.g. SEQ ID NO: 3 or SEQ ID NO: 4 is typically characterized using the NCBI Blast blastp with standard settings. The comparison of sequences and determination of percent identity between two amino acid sequences can also be accomplished with the program "BLAST 2 SEQUENCES (blastp)" (Tatusova et al., 1999) with the following parameters: Matrix BLOSUM62; Open gap 11 and extension gap 1 penalties; gap x_dropoff50; expect 10.0 word size 3; Filter: none.

Alternatively, sequence identity may be determined using the software GENEious with standard settings. Alignment results can be, e.g., derived from the Software Geneious (version R8), using the global alignment protocol with free end gaps as alignment type, and Blosum62 as a cost matrix.

Preferably, the first VEGF molecule of the column according to the first aspect of the invention or of the VEGF dimer molecule according to the second aspect of the invention has the amino acid sequence of SEQ ID NO: 3 and/or the second VEGF molecule of the column according to the first aspect of the invention or of the VEGF dimer molecule according to the second aspect of the invention has the amino acid sequence of SEQ ID NO: 4.

In a further preferred embodiment, the first VEGF molecule of the column according to the first aspect of the invention or of the VEGF dimer molecule according to the second aspect of the invention has at least 50% amino acid sequence identity in comparison to SEQ ID NO: 3, preferably at least 60% amino acid sequence identity in comparison to SEQ ID NO: 3, more preferably at least 80% amino acid sequence identity in comparison to SEQ ID NO: 3 and most preferably at least 90% amino acid sequence identity in comparison to SEQ ID NO: 3 and/or the second VEGF molecule of the column according to the first aspect of the invention or of the VEGF dimer molecule according to the second aspect of the invention has at least 50% amino acid sequence identity in comparison to SEQ ID NO: 4, preferably at least 60% amino acid sequence identity in comparison to SEQ ID NO: 4, more preferably at least 80% amino acid sequence identity in comparison to SEQ ID NO: 4 and most preferably at least 90% amino acid sequence identity in comparison to SEQ ID NO: 4.

The first and the second VEGF molecule of the column according to the first aspect of the invention or of the VEGF dimer molecule according to the second aspect of the invention may further be linked by a linker.

Alternatively, the VEGF dimer molecule may not comprise any linker. For example, the first and the second VEGF molecule of the column according to the first aspect of the invention or of the VEGF dimer molecule according to the second aspect of the invention may not be linked by a linker.

The term "linker" according to the present invention describes any spacer suitable to connect the first and the second VEGF molecule.

The linker may have a minimum length sufficient to allow flexibility of the first and/or second VEGF dimer relative to each other, e.g. leading to intermolecular multimerization of scVEGF, e.g leading to formation of scVEGF multimers, such as dimers and tetramers. The linker may have a length suitable to hamper intramolecular dimerization of VEGF-sequences.

The linker may comprise any material known to the person skilled in the art to be suitable for linking two protein molecules. For example, the linker may comprise any polymer of organic compounds known to the person skilled in the art, such as amino acids, nucleic acids, polysaccharides, polyethylene glycol and/or partial crosslinking, such as N-Hydroxysuccinimide (NHS). Preferably, the linker comprises amino acids and more preferably, the linker consists of amino acids. Moreover, the linker may be flexible or rigid.

For example, the nucleic acid sequences of the first and the second VEGF molecule may already be linked via the nucleic acid encoding the amino acid sequence of the linker and the whole construct may be expressed together as one VEGF dimer molecule (such as SEQ ID NO: 5) as described above.

The VEGF dimer molecule of the column according to the first aspect of the invention or according to the second aspect of the invention may have at least 50%, at least 55%, at least 60%, at least 65%, at least 70%, at least 75%, at least 80%, at least 85%, at least 90%, at least 95%, or 100% (e.g., at least 86%, at least 87%, at least 88%, at least 89%, at least 90%, at least 91%, at least 92%, at least 93%, at least 94%, at least 95%, at least 96%, at least 97%, at least 98%, at least 99% or 100% amino acid sequence identity in comparison to SEQ ID NO: 5. Preferably, the VEGF dimer molecule of the column according to the first aspect of the invention or according to the second aspect of the invention has 100% amino acid sequence identity in comparison to SEQ ID NO: 5.

Usually, the first and the second VEGF molecule may be linked by the linker, whereby the first end of the linker is connected to the C-terminal amino acid of the first VEGF molecule and the second end of the linker is connected to the N-terminal amino acid of the second VEGF molecule. Moreover, the first and the second VEGF molecule may be linked by the linker, whereby the first and/or second end of the linker are connected to an amino acid of the first and/or second VEGF molecule sequence that is not N-terminal or C-terminal.

In preferred embodiment, the first and the second VEGF molecule of the column according to the first aspect of the invention or of the VEGF dimer molecule according to the second aspect of the invention are linked by a linker.

If the linker comprises amino acids, the linker preferably has a length of 10 to 30 amino acids, preferably a length of 11 to 25 amino acids, more preferably a length of 12 to 20 amino acids, especially more preferably a length of 13 to 17 amino acids and most preferably a length of 14 amino acids

Moreover, the linker may comprise the sequence of SEQ ID NO: 6 (GSTSGSGKSSEGKG) or have a sequence identity of at least 50% amino acid sequence identity in comparison to SEQ ID NO: 6, preferably at least 70% amino acid sequence identity in comparison to SEQ ID NO: 6, more preferably at least 85% amino acid sequence identity in comparison to SEQ ID NO: 6 and most preferably at least 90% amino acid sequence identity in comparison to SEQ ID NO: 6. For example, it is possible to exchange single amino acids of SEQ ID NO: 6 for amino acids having the same or similar charge and/or size. Preferably, the linker comprises the sequence of SEQ ID NO: 6 and more preferably the linker consists of the sequence of SEQ ID NO: 6. Methods for determining the sequence identity of the linker in comparison to SEQ ID NO: 6 are well known to the person skilled in the art and comparable to those described above for determining sequence identity of the first and the second VEGF molecules to SEQ ID NOs: 3 and 4, respectively.

In a more preferred embodiment, the linker has a length of 10 to 30 amino acids, preferably a length of 11 to 25 amino acids, more preferably a length of 12 to 20 amino acids, especially more preferably a length of 13 to 17 amino acids and most preferably a length of 14 amino acids.

The VEGF dimer molecule of the column according to the first aspect of the invention or the VEGF dimer molecule according to the second aspect of the invention may be immobilized by a covalent bond to a matrix.

The matrix may be any molecule known to the person skilled in the art to be suitable for affinity chromatography of proteins. Usually, the matrix comprises chemical inert molecules. Especially, the molecules of the matrix may be suitable for clinical applications, such as apheresis. For example, they may have a low immunogenicity when applied in vivo.

Moreover, the molecules of the matrix generally allow covalent binding of the ligand VEGF dimer molecule. Therefore, a tag, such as a protein tag, may be connected to the VEGF dimer molecule and a matrix may be used that covalently binds this tag. Examples of suitable molecules of the matrix are well known to the person skilled in the art, such as sepharose, agarose and/or glutathione. Preferably, the matrix is sepharose or agarose, more preferably agarose. The matrix may further comprise a mixture of different molecules.

In another preferred embodiment, the VEGF dimer molecule of the column according to the first aspect of the invention or the VEGF dimer molecule according to the second aspect of the invention is immobilized by a covalent bond to a matrix.

In a third aspect, the invention relates to a method for preparing a column according to the first aspect comprising the steps of:

a) preparing by eukaryotic expression a vascular endothelial growth factor (VEGF) dimer molecule, wherein the VEGF dimer molecule comprises a first and a second VEGF molecule; and

b) immobilizing the dimer of step a) on a matrix.

Regarding the features of steps a) and b), all embodiments and features described above with respect to the column of the first aspect of the invention and the VEGF dimer molecule according to the second aspect of the invention also apply to this the third aspect.

Step a)

Methods for preparing a VEGF dimer molecule by eukaryotic expression are well known to the person skilled in the art and further described above. In particular, also the first and the second VEGF molecule and the linker are described above.

Step b)

Further, in step b) of the method for preparing a column according to the third aspect of the invention the VEGF dimer molecule is immobilized to the matrix by a covalent bond. Features concerning the matrix are also described above in the context of a preferred embodiment of the column of the first aspect of the invention and are applicable to the third aspect of the invention.

The term "immobilizing the dimer of step a) on a matrix" according to the present invention describes the fixation of a VEGF dimer molecule to the matrix. Methods for immobilizing a dimer on a matrix are well known to the person skilled in the art. For example, the VEGF dimer molecule may be immobilized on a matrix when the matrix is already loaded onto a column or before loading it onto a column. In the first example, the matrix is first loaded onto the column.

The VEGF dimer molecule may then be immobilized on that matrix by providing a solution comprising a VEGF dimer molecule and applying it to the matrix in the column. Due to specific interactions between the VEGF dimer molecule and the matrix, the VEGF dimer molecule usually stays in close proximity to the matrix or may bind to it, while the remaining substituents of the solution flow through the matrix and/or are removed by subsequent washing steps. Alternatively, in the second example, the matrix may be brought in contact with the VEGF dimer molecule leading to specific interactions between both of them. Subsequently, the matrix-VEGF dimer construct may be washed leading to removal of unspecifically binding components, but not of the specifically binding VEGF dimer molecules. Afterwards, the matrix with the immobilized VEGF dimer molecule may be loaded onto the column.

Moreover, in step b) the VEGF dimer molecule may be immobilized to the matrix by a covalent bond. Details of possible matrices that allow covalent binding of the VEGF dimer molecule are well known to the person skilled in the art and also described above for the column according to the first aspect of the invention.

The invention further relates to a column obtainable by the method according to the third aspect of the invention.

In a preferred embodiment, in step b) of the method for preparing a column according to the third aspect of the invention the VEGF dimer molecule is immobilized to the matrix by a covalent bond.

In a fourth aspect, the invention relates to the VEGF dimer molecule according to the second aspect for use in the treatment of preeclampsia characterized in that the VEGF dimer molecule is bound to a column for apheresis.

All embodiments and features described above with respect to the VEGF dimer molecule and the column as described in the first and/or second aspect of the invention also apply to this fourth aspect.

Moreover, the term "treatment of preeclampsia" according to the present invention comprises the treatment of all types of preeclampsia and all phases of this condition. For example, pregnant women may be treated as well as women, who recently gave birth and developed preeclampsia afterwards. Moreover, the treatment of preeclampsia also comprises the prophylactic treatment of pregnant women having a risk of developing preeclampsia, such as pregnant women suffering from antiphospholipid syndrome and/or hypertension or women having a sFlt-1/PIGF ratio, which is higher than the average sFlt-1/PIGF ratio in blood or serum in pregnant women, without showing symptoms of preeclampsia.

The term "apheresis" according to the present invention describes any extracorporeal medical therapy in which the blood or blood plasma of a person is passed through an apparatus that separates out one or more specific components, such as at least sFlt-1, and returns the remainder to the circulation. Thereby, beside the separation of sFlt-1, constituents bound to sFlt-1, such as VEGF and PIGF may be released into the blood and return to the circulation.

In general, apheresis methods and the required parameters are well known to the person skilled in the art. For example, an apheresis first comprises placing of a blood access. Therefore, needles are placed in peripheral veins of a patient. Single or double lumen catheters may also be used, for example in the internal jugular, subclavian of femoral veins. Usually, blood is then led from this first blood access into tubes or capillaries and pumped by a pump to an apheresis column, where sFlt-1 is separated from the blood and VEGF and PIGF are simultaneously released into the blood. The remaining constituents of the blood are then pumped by a second pump via further tubes or capillaries to a second blood access and returned to the patient's blood circulation. Moreover, before being introduced into the apheresis column, the blood may be separated and only the blood plasma may be applied to the apheresis column. Subsequently, the cleaned blood plasma may be combined with the blood cells that were separated previously during preparation of blood plasma and both may be returned to the patient's blood circulation.

The amount of blood plasma volume to be treated is usually the patient's estimated plasma volume (EPV). EPV is usually calculated using the following formula:

$$EPV = BW \times 1/13 \times (1 - Ht/100)$$

BW: Body weight (kg)
Ht: Hematocrit (%)

Further, an apheresis usually comprises measures preventing, interrupting and terminating coagulation. These measures are well known to the person skilled in the art. For example, anticoagulants, such as citrate (e.g. sodium citrate or acid-citrate-dextrose (e.g. ACD-A)) or heparin may be added to the blood or blood plasma during apheresis. Suitable anticoagulants and the required dosage is well known to the person skilled in the art and may further depend on the clinical and physical condition of the patient.

One column may be used in one apheresis treatment or repeatedly for up to 5, 10, 15, 20 or 25 apheresis treatment applications.

In addition to the VEGF dimer molecule as described for the column according to the first aspect of the invention or of the second aspect of the invention, the apheresis column may also comprise further ligands that may interact with sFlt-1. Moreover, the apheresis column may comprise further ligands for removing blood components other than sFlt-1 during one apheresis application. For example, a plasma exchange (e.g. for removing a liquid portion of blood comprising harmful substances and replacing the plasma with a replacement solution) may be performed or low density lipoprotein (LDL) may be removed.

Moreover, the present invention relates to a method of using a column comprising the VEGF dimer molecule according to the first aspect of the invention or the VEGF dimer molecule according to the second aspect of the invention to treat preeclampsia.

The invention also relates to a method for treating preeclampsia, comprising performing an apheresis using a column according to the first aspect of the invention or a VEGF dimer molecule according to the second aspect of the invention. All embodiments disclosed above also apply to these methods of the invention.

In a fifth aspect, the invention further relates to an expression vector comprising a nucleic acid sequence encoding the VEGF dimer molecule of the invention.

Suitable expression vectors are well known to the person skilled in the art. Preferably, the expression vector is suitable for the recombinant expression of a protein in eukaryotic cells.

For example, the expression vector may be a mammal-derived expression vector (e.g., pcDNA3 (manufactured by Invitrogen Corp.), pEGF-BOS (Mizushima and Nagata, 1990), pEF, and pCDM8), insect cell-derived expression vector (e.g., "Bac-to-BAC baculovirus expression system" (manufactured by Gibco BRL/Life Technologies, Inc.) and pBacPAK8), plant-derived expression vector (e.g., pMH1 and pMH2) or a yeast-derived expression vector (e.g., "Pichia Expression Kit" (manufactured by Invitrogen Corp.), pNV11, and SP-Q01). Preferably, the expression vector is the sleeping beauty transposon expression vector.

For the purpose of expression in animal cells such as CHO cells, COS cells, or NIH3T3 cells, the expression vector usually comprises a promoter for intracellular expression. Suitable promoters for intracellular expression are well known to the person skilled in the art. For example, SV40 promoter (Mulligan et al., 1979), MMLV-LTR promoter, EF1$\alpha$ promoter (Mizushima and Nagata, 1990), or CMV promoter may be used.

Moreover, the expression vector may further comprise a marker gene. Suitable marker genes are well known to the person skilled in the art. For example, the marker gene may be a drug resistance gene that is identifiable by a drug, such as neomycin, G418, etc., for transformed cells. Examples of vectors having such properties include pMAM, pDR2, pBK-RSV, pBK-CMV, pOPRSV, and pOP13.

All features describing the VEGF dimer molecule of the invention according to the second aspect of the invention also apply to the VEGF dimer molecule encoded by the nucleic acid sequence in the expression vector of the fifth aspect of the invention.

In a sixth aspect, the invention relates to a recombinant host cell line comprising the VEGF dimer molecule of the present invention, comprising the expression vector of the present invention and/or comprising a nucleic acid sequence encoding the VEGF dimer molecule of the present invention.

Suitable recombinant host cell lines are well known to the person skilled in the art. For example, the recombinant host cell line may be a eukaryotic cell line, such as a human cells line (e.g. HEK293T Epstein-Barr virus nuclear antigen (EBNA) cell line), a mouse cell line, a rat cell line, a hamster cell line, a yeast cell line or an insect cell line.

All features describing the VEGF dimer molecule of the invention according to the second aspect of the invention or describing the expression vector of the fifth aspect of the invention also apply to the VEGF dimer molecule or the expression vector of the recombinant host cell line of the sixth aspect of the invention.

The present invention further relates to a matrix or a bead comprising the VEGF dimer according to the second aspect of the invention.

All features described for the matrix of the column according to the first aspect of the invention or the matrix used in the third aspect of the invention also apply to the matrix according to the present invention.

The bead may be any bead known to the person skilled in the art to be suitable for affinity chromatography of proteins. Usually, the bead comprises chemical inert molecules. Especially, the bead may be suitable for clinical applications, such as apheresis. For example, the bead may have a low immunogenicity when applied in vivo.

The bead may further be any molecule known to be suitable to be used in a column according to the first aspect of the present invention or to bind to a matrix described or used in the present invention. Usually, the bead has a porous surface, whereby pore sizes suitable for affinity chromatography, such as apheresis, are well known to the person skilled in the art.

The bead may be of any material known to the person skilled in the art, such as agarose. The bead may further be of any size the known to the person skilled in the art to be suitable for the affinity chromatography of proteins, such as microbeads. Further, the bead may bind or interact with the VEGF dimer molecule as described for the first or second aspect of the invention. Moreover, the bead may bind or interact with the matrix used in the first or third aspect of the invention, which itself binds or interacts with the VEGF dimer molecule as described for the first or second aspect of the invention. In general, each binding VEGF dimer molecule or matrix molecule that is attached to the bead may be assumed to bind in a 1:1 ratio with the solute sample sent through the column that needs to be purified or separated.

In a seventh aspect, the invention relates to a method for separating sFlt-1 from blood and/or releasing VEGF from complexes with sFlt-1 into blood comprising incubating the column according to the first aspect of the invention or the VEGF dimer molecule according to the second aspect of the invention with the blood and separating sFlt-1 from the blood and/or releasing VEGF from complexes with sFlt-1 into the blood.

In the method according to the seventh aspect of the invention, the blood may be derived from a patient before separating sFlt-1 from the blood and/or releasing VEGF from complexes with sFlt-1 into the blood. Moreover, in the method according to the seventh aspect of the invention, the blood may be returned to the patient's blood circulation after separating sFlt-1 from the blood and/or releasing VEGF from complexes with sFlt-1 into the blood. Preferably, the method according to the seventh aspect of the invention is applied in the context of an apheresis, e.g. as described above in the context of the fourth aspect of the invention. All embodiments and features described above with respect to apheresis, e.g. as in the context of the fourth aspect of the invention, may also apply to this seventh aspect.

In the method according to the seventh aspect of the invention, sFlt-1 may be separated from blood and/or VEGF may be released from complexes with sFlt-1 into blood until the sFlt-1/PIGF ratio in the blood decreases, preferably, until the sFlt-1/PIGF ratio in the blood is <110, more preferably <85, especially more preferred <50 and most preferred <38.

Moreover, in the method according to the seventh aspect of the invention, sFlt-1 may be separated from blood and/or VEGF may be released from complexes with sFlt-1 into blood until the blood of the patient has amounts of sFlt-1 and/or VEGF in blood that are comparable to those of pregnant women without showing symptoms of preeclampsia.

Further, all embodiments and features described above with respect to the first, second, third and/or fourth aspect of the invention may also apply to this seventh aspect.

Moreover, the invention further relates to a method for separating sFlt-1 from blood and/or releasing VEGF from complexes with sFlt-1 into blood comprising incubating the column obtainable by the method according to the third aspect of the invention with the blood and separating sFlt-1 from the blood and/or releasing VEGF from complexes with sFlt-1 into the blood.

In an eight aspect, the invention relates to the use of the column according to the first aspect of the invention or the VEGF dimer molecule according to the second aspect of the invention for separating sFlt-1 from blood and/or releasing VEGF from complexes with sFlt-1 into blood.

Preferably, the use of the eight aspect of the invention is in the context of an apheresis, e.g. as described above in the context of the fourth aspect of the invention. All embodiments and features described above with respect to the first, second, third, fourth and/or seventh aspect of the invention may also apply to this eighth aspect.

Further, the invention relates to the use of the column obtainable by the method according to the third aspect of the invention for separating sFlt-1 from blood and/or releasing VEGF from complexes with sFlt-1 into blood.

The invention is not limited to the particular methodology, protocols and reagents described herein because they may vary. Furthermore, the terminology used herein is for the purpose of describing particular embodiments only and is not intended to limit the scope of the present invention. As used herein and in the appended claims, the singular forms "a", "an", and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, and materials are described herein.

The present invention is further illustrated by the following Figures and Examples, which are intended to explain, but not to limit the invention, and from which further features, embodiments and advantages may be taken. As such, the specific modifications discussed are not to be construed as limitations on the scope of the invention. It will be apparent to the person skilled in the art that various equivalents, changes, and modifications may be made without departing from the scope of the invention, and it is thus to be understood that such equivalent embodiments are to be included herein.

## Description of the figures

Figure 1: Molecular modelling of $scVEGF^{165}$ and negative staining electron microscopy. *A)* Schematic representation of the $scVEGF^{165}$ expression plasmid and potential modes of assembly into multimers bridged by intermolecular disulfide-bonds. *B)* Band and ribbon representation of two single chain $VEGF^{165}$-dimers assembling as tetrameric VEGF. The 14 amino acid linker is represented by red spheres. *C)* Visualization of purified recombinant $moVEGF^{165}$ and $scVEGF^{165}$ molecular structure by negative staining electron microscopy. $moVEGF^{165}$ molecules appear as dumbbell-shaped dimeric structures with a few monomeric dots. $scVEGF^{165}$ emerged as tetrameric structures and a few dumbbell-shaped single $scVEGF^{165}$ molecules. In the expanded image the contour of the respective molecule was outlined by automated image processing.

Figure 2: Binding characteristics of sFlt-1 capture molecules. *A)* Quantitative ELISA using VEGF-trap. Plot of absorbance 450nm versus VEGF-trap concentration on a semi logarithmic scale with [VEGF-trap] on the logarithmic scale. The curve defines the equilibrium dissociation constant (Kd). The lowest Kd belongs to $scVEGF^{165}$, indicating strongest interaction. Each value depicts calculated means of experimental triplicates (n=3). *B)* Calculated Kd values from three independent experimental replicates were summarized in order to compare the binding affinity between different VEGF/PIGF variants. $scVEGF^{165}$ reproducibly yields lowest Kd values, i.e. highest binding affinity. Plotted are means with SD from 3 independent experiments. *$p < 0.02$.

Figure 3: Generation of sFlt-1 capturing apheresis columns and evaluation of substrate matrices. *A)* Identification of ideal apheresis setup. Equal amounts of recombinant moPIGF, $moVEGF^{165}$, and $scVEGF^{165}$ were coupled to strepTactin XT, Cyanogen bromide activated sepharose, or amino-linked agarose resin. The binding affinity to sFlt-1 at a concentration of 1600 pg/ml in human serum was measured. sFlt-1 concentration in the flow through was determined by quantitative sFlt-1 ELISA. For all three coupled proteins, the measurements depicted the most significant sFlt-1 depletion when using the amino-linked agarose system. *B)* Longitudinal stability of the $scVEGF^{165}$ agarose column was assessed. Adsorption studies from human serum samples were performed at 1, 15, 30, 60, and 90 days. Within 90 days only a slight decrease in binding sFlt-1 reduction was noted.

Figure 4: Characterization of sFlt-1 capturing molecules employing human serum samples *A)* Concentration of sFlt-1 (pg/ml) in the sample (y-axis) after apheresis for all immobilized VEGF and PIGF variants as well as with the Flt-1 specific antibody (x-axis). *B)* Concentration of PIGF (pg/ml) in the sample (y-axis) after apheresis for all immobilized VEGF and PIGF variants as well as with the Flt-1 specific antibody (x-axis). *C)* Concentration of VEGF (pg/ml) in the sample (y-axis) after apheresis for all immobilized VEGF and PIGF variants as well as with the Flt-1 specific antibody (x-axis)..

Figure 5: Validation of sFlt-1 clearance and VEGF-/PIGF-release of $scVEGF^{165}$ columns. Serum samples of ten individual patients (x-axis) with preeclampsia were treated with $scVEGF^{165}$ apheresis. sFlt-1 (A), free PIGF (B), and VEGF (C) levels (y-axis) were measured before and after adsorption.

Figure 6: Characteristics of different VEGF- and PIGF-dependent sFlt-1 apheresis strategies and as compared to the antibody-based setup. Apheresis systems employing Flt-1 specific antibody reduce sFlt-1 levels but do not liberate endogenous PIGF or VEGF (far left panel). Adsorption columns containing PIGF retain sFlt-1 and liberate low quantities of endogenous PIGF but no endogenous VEGF. Monomeric $VEGF^{165}$ columns capture sFlt-1 and release PIGF and also VEGF. Enhanced binding characteristics of $scVEGF^{165}$ for sFlt-1 result in most efficient adsorption of sFlt-1 and competitive release of PIGF and VEGF (far right panel).

Figure 7: Purification of recombinant proteins shown by Coomassie stained sodium dodecyl sulfate polyacrylamide gel electrophoresis (SDS-PAGE). Coomassie staining of the supernatant of the stably transfected cells expressing the indicated proteins under induced expression upon doxycycline treatment. Initial medium containing the proteins was referred to as input. While recombinant proteins were not detected in the wash as well as flow-through, a clear purification profile of the proteins could be detected in the elution fraction.

Figure 8: Stability of immobilized VEGF/PIGF variants on agarose apheresis columns. Strep-tag specific antibody was employed to precipitate recombinant strep-tagged proteins from flow through of serum-treated $scVEGF^{165}$ columns. Precipitates were subjected to SDS-PAGE and immunoblot using strep-specific antibody. No strep-tagged protein was detected in the serum flow-through, indicating that no recombinant protein disengages from the column under apheresis conditions.

Figure 9: Evaluation of the functionality of recombinant sFlt-1, VEGF, and PIGF variants by SDS-PAGE followed by western blot. From top to bottom: specific antibody for phosphorylated ERK1/2, specific antibody for ERK1/2, specific antibody for glyceraldehyde 3-phosphate dehydrogenase (GAPDH). MAP-activation was detected by immunoblot analysis with a specific antibody for phosphorylated ERK1/ 2; equal loading and cell confluency was controlled by immunoblot for total ERK1/2 and GAPDH.

## Examples

## Material and Methods

### Cloning, recombinant protein expression and purification

For cloning scVEGF$^{165}$ and scPIGF, uncloned, double-stranded linear DNA fragments containing represented nucleotide sequence were customized and ordered in the form of GeneArt String DNA fragment (ThermoFisher scientific, Germany). The designed sequence bears two identical amino acid sequence of monomeric VEGF$^{165}$ except the second monomer lacking the signal peptide sequence. Two monomers are being connected to each other using a linker with amino acid sequence of GSTSGSGKSSEGKG (showed as underlined letters below). DNA fragments were amplified using primers (see Table 1) and cloned (using restriction enzymes NheI and BamHI) into the sleeping beauty transposon expression vector, carrying an N-terminal strep tag.

**Table 1:** Primer list used for cloning scVEGF$^{165}$ and scPIGF into expression vector.

| Primer type | Sequence | SEQ ID NO: |
|---|---|---|
| scVEGF$^{165}$ forward | 5'-ACAGCTAGCGCTCCTATGGCTGAAGGCGG-3' | 10 |
| scVEGF$^{165}$ reverse | 5'-TGTGGATCCCCGTCTGGGCTTATCGCAGC-3' | 11 |
| scPIGF forward | 5'-ACAGCTAGCCTGCCTGCTGTTCCTCCTC-3' | 12 |
| scPIGF reverse | 5'-TGTGGATCCCTCTACGAGGCACGGCGTCG-3' | 13 |

The GeneArt String DNA fragment sequences of scVEGF$^{165}$ and scPIGF are as follows (linker sequence underlined):

GeneArt String DNA fragment sequence of scVEGF$^{165}$ (SEQ ID NO: 7):

GCTAGCGCTCCTATGGCTGAAGGCGGAGGACAGAATCACCACGAGGTGGTCAAGTTCATGGACGTGTAC
CAGCGGAGCTACTGTCACCCCATCGAGACACTGGTGGACATCTTCCAAGAGTACCCCGACGAGATCGAG
TACATCTTCAAGCCCAGCTGCGTGCCCCTGATGAGATGTGGCGGCTGCTGCAATGACGAAGGCCTGGAA
TGTGTGCCCACCGAGGAATCCAACATCACCATGCAGATCATGCGGATCAAGCCCCACCAGGGCCAGCAT
ATCGGCGAGATGTCTTTCCTGCAGCACAACAAGTGCGAGTGCAGACCCAAGAAGGACCGGGCCAGACAA
GAGAATCCTTGCGGCCCTTGCAGCGAGCGGAGAAAGCACCTGTTTGTGCAGGACCCTCAGACCTGCAAG
TGCTCCTGCAAGAACACCGACAGCCGGTGCAAAGCCAGACAGCTGGAACTGAACGAGCGGACCTGCAGA
TGCGACAAGCCTAGAAGAGGCAGCACAAGCGGCAGCGGCAAAAGCTCTGAAGGCAAGGGAACGCGTGCC
CCAATGGCAGAAGGTGGCGGCCAGAACCACCATGAGGTCGTGAAGTTTATGGATGTCTATCAGCGGTCC
TACTGCCATCCTATCGAAACCCTGGTCGATATTTTTCAAGAGTATCCGGATGAGATTGAGTATATTTTC
AAACCCTCCTGTGTGCCGCTCATGCGCTGCGGCGGATGCTGTAATGATGAGGGACTTGAGTGCGTGCCA
ACCGAAGAGTCTAATATTACGATGCAGATTATGAGAATCAAACCGCATCAAGGGCAGCATATTGGGGAA
ATGAGCTTCCTCCAGCATAACAAATGTGAATGCCGGCCGAAGAAGGACAGAGCCCGGCAAGAAAACCCA
TGCGGCCCCTGTTCCGAGAGGCGGAAACATCTGTTCGTTCAAGATCCCCAGACCTGTAAATGTAGCTGT
AAAAACACCGACTCCAGGTGCAAGGCCCGGCAACTCGAGCTGAACGAGAGAACATGTCGCTGCGATAAG
CCCAGACGGGGATCCACA

GeneArt String DNA fragment sequence of scPIGF (SEQ ID NO: 8):

acagctagcCTGCCTGCTGTTCCTCCTCAACAATGGGCCCTGTCTGCCGGCAATGGCAGCTCTGAAGTT
GAGGTGGTGCCCTTCCAAGAAGTGTGGGGCAGAAGCTACTGCAGAGCCCTGGAAAGACTGGTGGACGTG
GTGTCTGAGTACCCCAGCGAGGTGGAACACATGTTCAGCCCTAGCTGCGTGTCCCTGCTGAGATGCACA
GGCTGTTGCGGCGACGAGAATCTGCACTGCGTGCCAGTGGAAACCGCCAACGTGACAATGCAGCTGCTG

AAAATCAGAAGCGGCGACAGACCCAGCTACGTGGAACTGACCTTCAGCCAGCACGTCCGCTGCGAGTGT
AGACCCCTGCGGGAAAAGATGAAGCCCGAGAGATGCGGAGATGCCGTGCCTAGAAGAGGCAGCACATCT
GGCTCTGGCAAGAGCAGCGAAGGCAAGGGACTTCCTGCTGTGCCACCACAGCAGTGGGCACTGAGTGCC
GGAAATGGCTCCTCTGAGGTGGAAGTCGTGCCTTTTCAAGAAGTCTGGGGACGCTCCTACTGTCGCGCT
CTTGAGAGACTGGTCGATGTCGTCAGCGAGTACCCCTCCGAAGTCGAGCACATGTTTTCCCCATCCTGT
GTGTCTCTGCTGCGGTGTACCGGATGCTGCGGGGATGAGAACCTGCATTGTGTGCCTGTCGAGACAGCC
AATGTCACCATGCAGCTCCTCAAGATCAGATCCGGCGATCGGCCCTCCTACGTCGAGCTGACATTTTCT
CAGCACGTTCGATGCGAATGCCGGCCTCTGCGCGAGAAAATGAAGCCTGAACGCTGTGGCGACGCCGTG
CCTCGTAGAggatccaca

Protein sequence scPIGF (linker underlined) (SEQ ID NO: 9):

TASLPAVPPQQWALSAGNGSSEVEVVPFQEVWGRSYCRALERLVDVVSEYPSEVEHMFSPSCVSLLRCT
GCCGDENLHCVPVETANVTMQLLKIRSGDRPSYVELTFSQHVRCECRPLREKMKPERCGDAVPRRGSTS
GSGKSSEGKGLPAVPPQQWALSAGNGSSEVEVVPFQEVWGRSYCRALERLVDVVSEYPSEVEHMFSPSC
VSLLRCTGCCGDENLHCVPVETANVTMQLLKIRSGDRPSYVELTFSQHVRCECRPLREKMKPERCGDAV
PRRGST

For the production of recombinant protein stable HEK293T Epstein-Barr virus nuclear antigen (EBNA) cell lines were generated employing the sleeping beauty transposon system and the protein was purified as previously described (Agarwal et al., 2012). Briefly, the vectors were transfected into the HEK293T EBNA cells using FuGENE® HD transfection reagent (Promega GmbH, Madison, USA) for the duration of 3 days with fetal bovine serum (FBS)- free medium. Supernatant of the cells was harvested on the following days, filtered and the proteins carrying the Strep tag were purified via Strep-Tactin®XT (IBA Lifesci-ence, Göttingen, Germany) resin at room temperature (RT). The proteins were then eluted by Biotin containing Tris-buffered saline (TBS)- buffer (IBA Lifescience, Göttingen, Germany), and the aliquots were stored at -80°C.

Negative stain electron microscopy

The structure of monomeric and dimeric VEGF was visualized by negative staining electron microscopy as described previously (Bober et al., 2010). Briefly, samples (usual concentrations 10 - 20 nM) were incubated on carbon-coated grids for 1 min, washed and then stained with 0.75% uranyl formate for 1 min. The grids were rendered hydrophilic by glow discharge at low pressure in air. Specimens were examined in a Philips/FEI CM 100 TWIN transmission electron microscope operated at 60 kV accelerating voltage. Images were recorded with a side-mounted Olympus Veleta camera with a resolution of 2048 x 2048 pixels (2k x 2K) and the ITEM acquisitions software.

Human umbilical vein endothelial cell (HUVEC) cell culture and mitogen treatment

HUVEC passage 3 were cultured on collagen II coated 6 well plate with Endopan 3 kit medium containing 5% FBS, hydrocortisone, VEGF, hFGF-B, R3-IGF-1, ascorbic acid, hEGF, GA-1000 (Pan biotech, Bavaria, Germany) until they reached 80% confluency. The cells were starved with 3% FBS and no additive growth factor supplement for 24 hours. On the next day, the cells were stimulated with the recombinant proteins as indicated. After incubation for 10 minutes at 37°C, the cells were directly lysed on the plate using 30 μl 2x Laemmli buffer, and loaded on a sodium dodecyl sulfate (SDS) polyacrylamide gel, followed by western blot.

Solid phase enzyme-linked immunosorbent assay (ELISA) style assay

ELISA style binding assays were performed as described previously (Agarwal et al., 2012). In brief, 10 μg/ml of recombinant VEGF, PIGF and their mutant variants were individually immobilized together with bovine serum albumin (BSA) on 96 well plate (Transparente Immuno Standardmodule, Thermo scientific, Denmark), while matrix loaded only with BSA served as a control. A binding saturation curve was generated by applying increasing concentrations of VEGFR1/R2 trap ranging from 0,1 to 750 nM, followed by detection of the ligand-receptor complex using a horseradish peroxidase (HRP)-conjugated antibody against the mouse Fc domain of VEGFR1/R2 trap. Luminescence was quantified at optical density (OD) 450 nm using a by spectrophotometer (Thermo ScientificMultiskan GO, Finland).

ELISA measurements

Human sFlt-1, free VEGF, and PIGF levels were quantified using specific commercial ELISA kits (R&D Systems, Minneapolis, USA). The assays were performed as specified by the manufacturer. Fluorescence was quantified using a spectrophotometer (Thermo ScientificMul-tiskan GO, Finland). Data was analyzed with GraphPad prism 7 software (GraphPad Soft-ware Inc., La Jolla, CA, USA).

Generation of specific VEGF/PIGF columns

The immobilization of the recombinant protein on Strep-Tactin®XT matrix (IBA Lifescience, Got-tingen,Germany) was performed on Polyprep® chromatography columns (Bio-Rad laboratories, USA) according to the manufacturers' instructions.

The recombinant proteins containing a Strep tag, were incubated with the recommended amount of resin in TRIS 50 mM, NaCl 150mM, pH=8 overnight.
For immobilization of recombinant protein on Cyanogen bromide activated resin (Merck, Germany) on Polyprep® chromatography columns (Bio-Rad laboratories, USA), the proteins where dialyzed in coupling buffer containing 100 mM $NaHCO_3$, 500 mM NaCl, pH=8.3 for 2 days. The beads were activated with 30 ml cold 1 mM HCl for 15 min. The recommended amount of resin was incubated with the dialyzed proteins overnight at 4°C. The unbound surface of the beads was blocked by Tris-HCl 0.1 M for 2 hours at RT.
For the immobilization of recombinant protein on an agarose matrix (AminoLink™ Plus Immobilization Kit, Thermo Fisher scientific, USA), the manufacturers' instruction was followed. Briefly, the recombinant proteins were incubated in coupling buffer pH=10, added to the resin and incubated for 4 hours with 50 mM $NaCNBH_3$ in coupling buffer pH=7.2. Blocking of the unbound surface of the resin was performed by incubation with 50 mM $NaCNBH_3$ in quenching buffer.
In all cases, the protein concentrations were measured before and after the coupling to determine the coupling efficiency. An equal concentration of around 500 mg/ml from each recombinant protein was used for immobilization to the corresponding resin.
For the assessment of the longitudinal stability of the scVEGF165 agarose column, 1.8 ml of VEGF coupled agarose resin was generated, divided into 6 aliquots of 300 µl and stored at 4°C. Adsorption studies were performed after 1, 15, 30, 60, and 90 days.

Precipitation of Strep-tagged proteins from flow through

Strep-tagged proteins were precipitated from the flow through after apheresis treatment of serum samples by adding Strep-Tactin®XT (IBA Lifescience) beads. For the positive control, recombinant strep-tagged VEGF was added to the serum flow through. After washing the beads twice with TRIS 50 mM, NaCl 150 mM, pH=8, 30 µl 2 x Laemmli buffer was directly added to the beads followed by SDS polyacrylamide gel electrophoresis, and immunoblot blot using Strep-tag specific antibody.

Ethics approval and informed consent

Written informed consent was obtained from all participants according to protocols 10-238 and 09-258 as reviewed and approved by the ethics committee of the University Hospital Cologne. Preeclampsia was defined as new onset of hypertension >140/90 mmHg, proteinuria >0.3 g/g Creatinine, and sFlt-1/PIGF > 85 at EGA ≤32 weeks.

## Example 1 - Molecular modelling and structural analysis of single chain $VEGF^{165}$ dimers

Intermolecular disulfide bonds stabilize the structure of VEGF and PIGF homodimers and unfold the receptor binding sites. These unique properties could be utilized to engineer higher order multimers of VEGF and PIGF with enhanced binding affinity to sFlt-1.

Expression constructs containing a $VEGF^{165}$ lacking the N-terminal sequence followed by a short inert 14 amino acid linker and a second $VEGF^{165}$ lacking the N-terminal signal peptide (single chain VEGF dimers = $scVEGF^{165}$) equipped with a cleavable Strep-tag® for purification (Fig. 1A) were designed (SEQ ID NO: 7). It was speculated that the short 14-amino acid linker hampered assembly of $scVEGF^{165}$ in a 40 kDa dimer. In contrast, an open structure of two or more $scVEGF^{165}$ molecules linked by intermolecular disulfide bonds would allow formation of tetramers, or multimeric chains of VEGF. Molecular modelling approaches of the tetrameric quaternary assembly of $scVEGF^{165}$ were based on structural restraints of the 14-amino acid linker and negative stain electron micrographs (Fig. 1B+C).

Monomeric wildtype $VEGF^{165}$ ($moVEGF^{165}$, full-length $VEGF^{165}$ lacking the N-terminal sequence), and $scVEGF^{165}$ constructs were cloned as detailed above (see also Table 1), expressed in human embryonic kidney cells (HEK 293T), and the protein was purified under physiologic conditions using Strep-Tactin® technology (Fig. 7). The constructs were biologically functional as assessed in experiments investigating mitogen-activated protein kinase (MAP)-kinase activation in HUVEC cells in culture (Fig. 9). Activation of map kinase pathway can be detected after treatment with recombinant VEGF and PLGF variants. Co-treatment of VEGF and sFlt-1 abrogates MAP-activation (Fig. 9).

To visualize the quaternary molecular structure of $scVEGF^{165}$ in comparison to $moVEGF^{165}$, negative staining electron microscopy was employed (Fig. 1C). As expected for monomeric expressed VEGF, $moVEGF^{165}$ assembled in dumbbell-shaped dimeric complexes with a few monomeric molecules represented as single dots (left panel Fig. 1C). In striking contrast, $scVEGF^{165}$ appeared as uniformly ordered cloverleaf-shaped structures representing complexes of $scVEGF^{165}$ in a 2:2 configuration, i.e. tetramers of $VEGF^{165}$.

In addition to $moVEGF^{165}$ and $scVEGF^{165}$, monomeric PIGF (moPIGF) and single chain PIGF dimers (scPIGF) constructs were generated and expressed and tested as previously described (Fig. 7 top right and bottom right).

Subsequently, binding characteristics of $moVEGF^{165}$, $scVEGF^{165}$, moPIGF and scPIGF were explored. To screen for strong interactors, the binding affinity and static binding capacity of VEGF and PIGF constructs to VEGF-trap were quantified as the equilibrium dissociation constant (Kd) by ELISA based serial dilution experiments and protein loading in high excess, respectively. The VEGF-Trap as used in the present application is a chimeric protein containing the second binding domain of the VEGFR-1 receptor and the third domain of the VEGFR-2 receptor fused to the Fc segment of a human IgG backbone resulting in a very high VEGF binding affinity (Kd≈1 pM).

As expected from previous reports, $moVEGF^{165}$ displayed higher affinity (2.6 fold) as compared to moPIGF (Christinger et al., 2004). Strikingly, $scVEGF^{165}$ however showed 11% higher affinity as compared to $moVEGF^{165}$. Interestingly, affinity of scPIGF was not significantly different compared to moPIGF (Fig. 2A+B).

Results: It is a very surprising finding that the binding affinity of $scVEGF^{165}$ to VEGF-trap is 11% higher in comparison to that $moVEGF^{165}$, especially in light of the fact that the binding affinity of scPIGF in comparison to moPIGF is approximately equal. Further, the surprisingly large variation in binding capacity found in the different constructs is remarkable.

## Example 2- Evaluation of substrate matrices and stability of sFlt-1 capturing apheresis columns

To analyze coupling efficiency of recombinant proteins to different resins, the recombinant protein concentration before and after coupling to the resin was determined. The results are shown in Table 2.

**Table 2:** Representative data showing recombinant protein concentration before and after coupling to resin, to determine coupling efficiency of recombinant proteins to different resin.

| Protein name | [Protein](μg/ ml) before coupling | [Protein](μg/ml) after coupling | Percentage |
|---|---|---|---|
| $moVEGF^{165}$ coupled agarose | 519.1 | 0.192 | 99.96 |
| $moVEGF^{165}$ coupled CnBr | 518.9 | 0.124 | 99.97 |
| $moVEGF^{165}$ coupled StrepTactin | 518.5 | 0.187 | 99.96 |
| moPIGF coupled agarose | 487.9 | 0.161 | 99.967 |
| moPIGF coupled CnBr | 490 | 0.117 | 99.97 |
| moPIGF coupled StrepTactin | 489.2 | 0.158 | 99.96 |
| $moVEGF^{165}$ coupled agarose | 513 | 0.113 | 99.977 |
| $scVEGF^{165}$ coupled agarose | 538 | 0.189 | 99.96 |
| VEGF DR coupled agarose | 523 | 0.154 | 99.97 |
| moPLGF coupled agarose | 512 | 0.139 | 99.97 |
| scPIGF coupled agarose | 524 | 0.143 | 99.97 |
| Flt-1 antibody coupled agarose | 543 | 0.182 | 99.96 |

To optimize the dynamic binding characteristics of the apheresis columns, sFlt-1 adsorption from human serum by immobilized moPIGF and moVEGF$^{165}$ was tested using Streptactin XT®, Cyanogen bromide activated sepharose or aldehyde-activated agarose for immobilization of the ligand. Significant adsorption of sFlt-1 was noted for all resins on both moPIGF- as well as moVEGF$^{165}$-based columns. However, the largest reduction of 86.16 % sFlt-1 in a single run was achieved with columns where scVEGF$^{165}$ was immobilized on agarose matrix (Fig. 3A). Stability of the scVEGF$^{165}$-based agarose column was assessed at 1, 15, 30, 60, and 90 days after generation and no significant loss of affinity noted (Fig. 3B).

Results: Binding of a ligand to an agarose matrix led for all ligands (moPIGF, moVEGF$^{165}$ and scVEGF$^{165}$) to the largest adsorption of sFlt-1 from human serum in comparison to binding to a Streptactin XT® matrix or a sepharose matrix. Moreover, sFlt-1 adsorption from human serum was significantly higher for scVEGF$^{165}$ in comparison to moPIGF or moVEGF$^{165}$ (with moVEGF$^{165}$ having a slightly better sFlt-1 adsorption from human serum in comparison to moPIGF). The largest reduction of 86.16 % sFlt-1 in a single run was achieved with columns where scVEGF$^{165}$ was immobilized on agarose matrix. Moreover, the examination of longitudinal stability of scVEGF$^{165}$ agarose column revealed only a slight decrease in binding sFlt-1.

## Example 3 - Characterization of sFlt-1 capturing ligands

In a next step, the efficacy of sFlt-1 reduction from patient serum was assessed for the different VEGF- and PIGF-variants immobilized on agarose apheresis columns.

Mini columns carrying aldehyde-activated agarose were equally loaded with VEGF- or PIGF-variants (Table 2). In addition, one antibody-based apheresis column was generated to provide a reference control. sFlt-1, VEGF-, and PIGF-concentrations were determined in a serum sample of a patient with preeclampsia using commercial ELISA kits before and after single runs over a control column or columns equipped with the respective VEGF- or PIGF-variant or Flt-1 specific antibody. Whereas no difference between sFlt-1-, PIGF-, and VEGF-concentrations was noted after running the sample over the control column, sFlt-1 levels were reduced by varying amounts depending on the specific ligand (Fig. 4A). With moVEGF$^{165}$ immobilized on the column 77.15% reduction of sFlt-1 was achieved in a single passage of patient serum, which equaled reduction achieved by the antibody column. In contrast and as expected (Christinger et al., 2004) due to the lower binding affinity and capacity of moPIGF as compared to moVEGF$^{165}$, adsorption with the moPIGF column only yielded 46.18% reduction of sFlt-1. For moVEGF columns liberation of PIGF and VEGF was noted, whereas for moPIGF columns only release of PIGF but no release of VEGF was detected and the antibody column released neither VEGF nor PIGF (Fig. 4B+C).

Strikingly, in the adsorption experiments employing patient serum, the modified single chain VEGF-dimers (scVEGF$^{165}$) boasted sFlt-1 reduction to 89.87% in a single run (Fig. 4A), while at the same time, releasing VEGF and PIGF in large amounts (Fig. 4B+C). Interestingly, for PIGF the optimized multimerization strategy did not affect sFlt-1 binding and PIGF release to the same extent (Fig. 4A). Only minor enhancement of sFlt-1 binding and no significant difference in PIGF release was documented for the scPIGF column as compared to the moPIGF column (Fig. 4A+B). Expectedly, as for moPIGF the scPIGF columns did not release endogenous VEGF (Fig. 4C).

The notion that increased VEGF or PIGF levels in the flow through might represent leakage of recombinant protein from the column was invalidated by purification of Strep-tagged proteins from the flow through of columns carrying Strep-tagged VEGF or PIGF after passage of serum samples. For all constructs tested, no leaked protein was detected in western blot analysis employing Strep-specific primary antibody (Fig. 9).

Results: All columns with immobilized VEGF and PIGF variants as well as with the Flt-1 specific antibody reduced sFlt-1 in the sample. The reduction of sFlt-1 was greatest for scVEGF$^{165}$ columns ($p<0.0001$), all other VEGF and PIGF variants as well as the Flt-1 specific antibody reduced sFlt-1 less efficiently (Fig. 4A). Apheresis with VEGF and PIGF columns resulted in release of PIGF most pronounced for scVEGF$^{165}$ ($p<0.0001$), while apheresis with moVEGF$^{165}$ only resulted in a release of PIGF half that much of scVEGF$^{165}$. Apheresis with sFlt-1 antibody did not result in a significant PIGF release (Fig. 4B). VEGF release was only present in apheresis columns containing VEGF variants, again most pronounced for scVEGF$^{165}$ ($p<0.0001$). Apheresis of serum samples using columns with PIGF variants and Flt-1 specific antibody did not result in VEGF liberation (Fig. 4C).

## Example 4 - Validation of scVEGF$^{165}$-based apheresis in independent patient samples

The yield of sFlt-1 reduction and VEGF-/PIGF-release by scVEGF$^{165}$ columns was substantiated in serum samples of 10 independent patients at different gestational ages with clinically suspected preeclampsia and high sFlt-1/PIGF ratios.

Results: In all patient samples, sFlt-1 reduction of 88% (mean) (median 92.2%; SD 5.27) (Fig. 5A), PIGF release of 20-fold (mean) compared to the initial levels (median 14.57 fold; SD 5.40) (Fig. 5B) as well as VEGF release of 9.07 fold (mean) compared to the initial levels (median 8.74.; SD 4.15) (Fig. 5C) was achieved in a single run over the scVEGF$^{165}$ column.

Apheresis systems employing Flt-1 specific antibody reduce sFlt-1 levels but do not liberate endogenous PIGF or VEGF (Fig. 6A). Adsorption columns containing PIGF retain sFlt-1 and liberate low quantities of endogenous PIGF but not endogenous VEGF (Fig. 6B). Monomeric $VEGF^{165}$ columns capture sFlt-1 and release larger quantities of PIGF and also VEGF (Fig. 6C). Enhanced binding characteristics of $scVEGF^{165}$ for sFlt-1 result in most efficient adsorption of sFlt-1 and competitive release of PIGF and VEGF leading to restitution of the angiogenic balance in preeclampsia (Fig. 6D).

In general, the unique characteristic of $scVEGF^{165}$-based apheresis compared to antibody-mediated apheresis and moVEGF- or PIGF-based approaches is the enhanced affinity for sFlt-1 and thus the capability to efficiently release VEGF and PIGF (Fig. 6).

**References**

Sibai BM. Prevention of preeclampsia: a big disappointment. Am J Obstet Gynecol. 1998; 179:1275-1278.

Redman CW. Latest Advances in Understanding Preeclampsia. Science. 2005;308:1592-1594.

Walker JJ. Pre-eclampsia. Lancet. 2000;356:1260-1265.

Maynard SE, Min J-Y, Merchan J, Lim K-H, Li J, Mondal S, Libermann TA, Mor-gan JP, Sellke FW, Stillman IE, Epstein FH, Sukhatme VP, Karumanchi SA. Ex-cess placental soluble fms-like tyrosine kinase 1 (sFlt1) may contribute to endo-thelial dysfunction, hypertension, and proteinuria in preeclampsia. J Clin Invest. 2003;111:649-658.

Levine RJ, Maynard SE, Qian C, Lim K-H, England LJ, Yu KF, Schisterman EF, Thadhani R, Sachs BP, Epstein FH, Sibai BM, Sukhatme VP, Karumanchi SA. Circulating angiogenic factors and the risk of preeclampsia. N Engl J Med. 2004;350:672-683.

ACOG Practice Bulletin No. 202: Gestational Hypertension and Preeclampsia. Obstetrics & Gynecology. 2019;133:e1-e25.

Makris A, Yeung KR, Lim SM, Sunderland N, Heffernan S, Thompson JF, Ili-opoulos J, Killingsworth MC, Yong J, Xu B, Ogle RF, Thadhani R, Karumanchi SA, Hennessy A. Placental Growth Factor Reduces Blood Pressure in a Utero-placental Ischemia Model of Preeclampsia in Nonhuman Primates. Hyperten-sion. 2016;67:1263-1272.

Li Z, Zhang Y, Ying Ma J, Kapoun AM, Shao Q, Kerr I, Lam A, O'Young G, Sannajust F, Stathis P, Schreiner G, Karumanchi SA, Protter AA, Pollitt NS. Re-combinant vascular endothelial growth factor 121 attenuates hypertension and improves kidney damage in a rat model of preeclampsia. Hypertension. 2007;50:686-692.

Eddy AC, Bidwell III GL, George EM. Pro-angiogenic therapeutics for preeclampsia. Biol Sex Differ. 2018; 9: 36.

Turanov AA, Lo A, Hassler MR, Makris A, Ashar-Patel A, Alterman JF, Coles AH, Haraszti RA, Roux L, Godinho BMDC, Echeverria D, Pears S, Iliopoulos J, Shanmugalingam R, Ogle R, Zsengeller ZK, Hennessy A, Karumanchi SA, Moore MJ, Khvorova A. RNAi modulation of placental sFLT1 for the treatment of preeclampsia. Nature Biotechnology. 2018;36:1164-1173.

Fan X, Rai A, Kambham N, Sung JF, Singh N, Petitt M, Dhal S, Agrawal R, Sut-ton RE, Druzin ML, Gambhir SS, Ambati BK, Cross JC, Nayak NR. Endometrial VEGF induces placental sFLT1 and leads to pregnancy complications. Journal of Clinical Investigation. 2014; 124:4941-4952.

Thadhani R, Kisner T, Hagmann H, Bossung V, Noack S, Schaarschmidt W, Jank A, Kribs A, Cornely OA, Kreyssig C, Hemphill L, Rigby AC, Khedkar S, Lindner TH, Mallmann P, Stepan H, Karumanchi SA, Benzing T. Pilot Study of Extracorporeal Removal of Soluble Fms-Like Tyrosine Kinase 1 in Preeclampsia. Circulation. 2011;124:940-950.

Thadhani R, Hagmann H, Schaarschmidt W, Roth B, Cingoez T, Karumanchi SA, Wenger J, Lucchesi KJ, Tamez H, Lindner T, Fridman A, Thome U, Kribs A, Danner M, Hamacher S, Mallmann P, Stepan H, Benzing T. Removal of Soluble Fms-Like Tyrosine Kinase-1 by Dextran Sulfate Apheresis in Preeclampsia. J Am Soc Nephrol. 2016;27:903-913.

Trapiella-Alfonso L, Alexandre L, Fraichard C, Pons K, Dumas S, Huart L, Gaucher JF, Hebert-Schuster M, Guibourdenche J, Fournier T, Vidal M, Broutin I, Lecomte-Raclet L, Malaquin L, Descroix S, Tsatsaris V, Gagey-Eilstein N, Lecarpentier E. VEGF (Vascular Endothelial Growth Factor) Functionalized Magnetic Beads in a Microfluidic Device to Improve the Angiogenic Balance in Preeclampsia. Hypertension. 2019 Jul;74(1):145-153. doi: 10.1161/HYPERTENSIONAHA.118.12380. Epub 2019 May 13.

Jain A, Cheng K. The principles and applications of avidin-based nanoparticles in drug delivery and diagnosis. Journal of Controlled Release. 2017;245:27-40.

Claffey KP, Senger DR, Spiegelman BM. Structural requirements for dimeriza-tion, glycosylation, secretion, and biological function of VPF/VEGF. Biochimica et Biophysica Acta (BBA) - Protein Structure and Molecular Enzymology. 1995;1246:1-9.

Peretz D, Gitay-Goren H, Safran M, Kimmel N, Gospodarowicz D, Neufeld G. Glycosylation of vascular endothelial growth factor is not required for its mito-genic activity. Biochemical and Biophysical Research Communications. 1992;182:1340-1347.

Kuriakose A, Chirmule N, Nair P. Immunogenicity of Biotherapeutics: Causes and Association with Posttranslational Modifications. Journal of Immunology Research. 2016;2016:1-18.

Tatusova et al. FEMS Microbiol. Lett. 1999; 174: 247-250.

Mizushima and Nagata, pEF-BOS, a powerful mammalian expression vector. Nucleic Acids Res. (1990) 18(17), 5322.

Mulligan RC, Howard BH, Berg P. Synthesis of rabbit beta-globin in cultured monkey kidney cells following infection with a SV40 beta-globin recombinant genome. Nature. 1979; 277(5692):108-14.

Agarwal P, Zwolanek D, Keene DR, Schulz J-N, Blumbach K, Heinegård D, Zaucke F, Paulsson M, Krieg T, Koch M, Eckes B. Collagen XII and XIV, New Partners of Cartilage Oligomeric Matrix Protein in the Skin Extracellular Matrix Suprastructure. Journal of Biological Chemistry. 2012;287:22549-22559.

Bober M, Enochsson C, Collin M, Morgelin M. Collagen VI Is a Subepithelial Adhesive Target for Human Respiratory Tract Pathogens. Journal of Innate Immunity. 2010;2:160-166.

Christinger HW, Fuh G, de Vos AM, Wiesmann C. The Crystal Structure of Pla-cental Growth Factor in Complex with Domain 2 of Vascular Endothelial Growth Factor Receptor-1. Journal of Biological Chemistry. 2004;279:10382-10388.

**Claims**

1. A column comprising a vascular endothelial growth factor (VEGF) dimer molecule comprising a first and a second VEGF molecule.

2. A VEGF dimer molecule comprising a first and a second VEGF molecule, wherein the first and/or the second VEGF molecule have a length of 122 to 250 amino acids.

3. The column according to claim 1 or the VEGF dimer molecule according to claim 2, wherein the VEGF dimer molecule is expressed by a eukaryotic cell.

4. The column according to any one of claims 1 or 3 or the VEGF dimer molecule according to claims 2 to 3, wherein the first and the second VEGF molecule are identical.

5. The column according to any one of claims 1 or 3 to 4 or the VEGF dimer molecule according to any one of claims 2 to 4, wherein the first and/or the second VEGF molecule lack the N-terminal signal peptide.

6. The column according to any one of claims 1 or 3 to 5 or the VEGF dimer molecule according to any one of claims 2 to 5, wherein the first and/or the second VEGF molecule have a length of 122 to 250 amino acids, preferably 125 to 225 amino acids, more preferably 140 to 200 amino acids, even more preferably 150 to 180 amino acids, especially more preferably 160 to 170 amino acids and most preferably 165 amino acids.

7. The column according to any one of claims 1 or 3 to 6 or the VEGF dimer molecule according to any one of claims 2 to 6, wherein the first VEGF molecule has at least 50% amino acid sequence identity in comparison to SEQ ID NO: 3, preferably at least 60% amino acid sequence identity in comparison to SEQ ID NO: 3, more preferably at least 80% amino acid sequence identity in comparison to SEQ ID NO: 3 and most preferably at least 90% amino acid sequence identity in comparison to SEQ ID NO: 3 and/or, wherein the second VEGF molecule has at least 50% amino acid sequence identity in comparison to SEQ ID NO: 4, preferably at least 60% amino acid sequence identity in comparison to SEQ ID NO: 4, more preferably at least 80% amino acid sequence identity in comparison to SEQ ID NO: 4 and most preferably at least 90% amino acid sequence identity in comparison to SEQ ID NO: 4.

8. The column according to any one of claims 1 or 3 to 7 or the VEGF dimer molecule according to any one of claims 2 to 7, wherein the first and the second VEGF molecule are linked by a linker.

**9.** The column according to any one of claim 8 or the VEGF dimer molecule according to claim 8, wherein the linker has a length of 10 to 30 amino acids, preferably a length of 11 to 25 amino acids, more preferably a length of 12 to 20 amino acids, especially more preferably a length of 13 to 17 amino acids and most preferably a length of 14 amino acids.

**10.** The column according to any one of claims 1 or 3 to 9 or the VEGF dimer molecule according to any one of claims 2 to 9, wherein the VEGF dimer molecule is immobilized by a covalent bond to a matrix.

**11.** A method for preparing a column according to any one of claims 1 or 3 to 10 comprising the steps of:

a) preparing by eukaryotic expression a vascular endothelial growth factor (VEGF) dimer molecule comprising a first and a second VEGF molecule; and
b) immobilizing the dimer of step a) on a matrix.

**12.** A method for preparing a column according to claim 11, wherein in step b) the VEGF dimer molecule is immobilized to the matrix by a covalent bond.

**13.** The VEGF dimer molecule according to any one of claims 2 to 10 for use in the treatment of preeclampsia, **characterized in that** the VEGF dimer molecule is bound to a column for apheresis.

**14.** An expression vector comprising a nucleic acid sequence encoding the VEGF dimer molecule according to any one of claims 2 to 10.

**15.** A recombinant host cell line comprising the VEGF dimer molecule according to any one of claims 2 to 10, comprising the expression vector according to claim 15 and/or comprising a nucleic acid sequence encoding the VEGF dimer molecule according to any one of claims 2 to 10.

**16.** A method for separating sFlt-1 from blood and/or releasing VEGF from complexes with sFlt-1 into blood comprising incubating the column according to any one of claims 1 or 3 to 9 or the VEGF dimer molecule according to any one of claims 2 to 9 with the blood and separating sFlt-1 from the blood and/or releasing VEGF from complexes with sFlt-1 into the blood.

**17.** Use of the column according to any one of claims 1 or 3 to 9 or the VEGF dimer molecule according to any one of claims 2 to 9 for separating sFlt-1 from blood and/or releasing VEGF from complexes with sFlt-1 into blood.

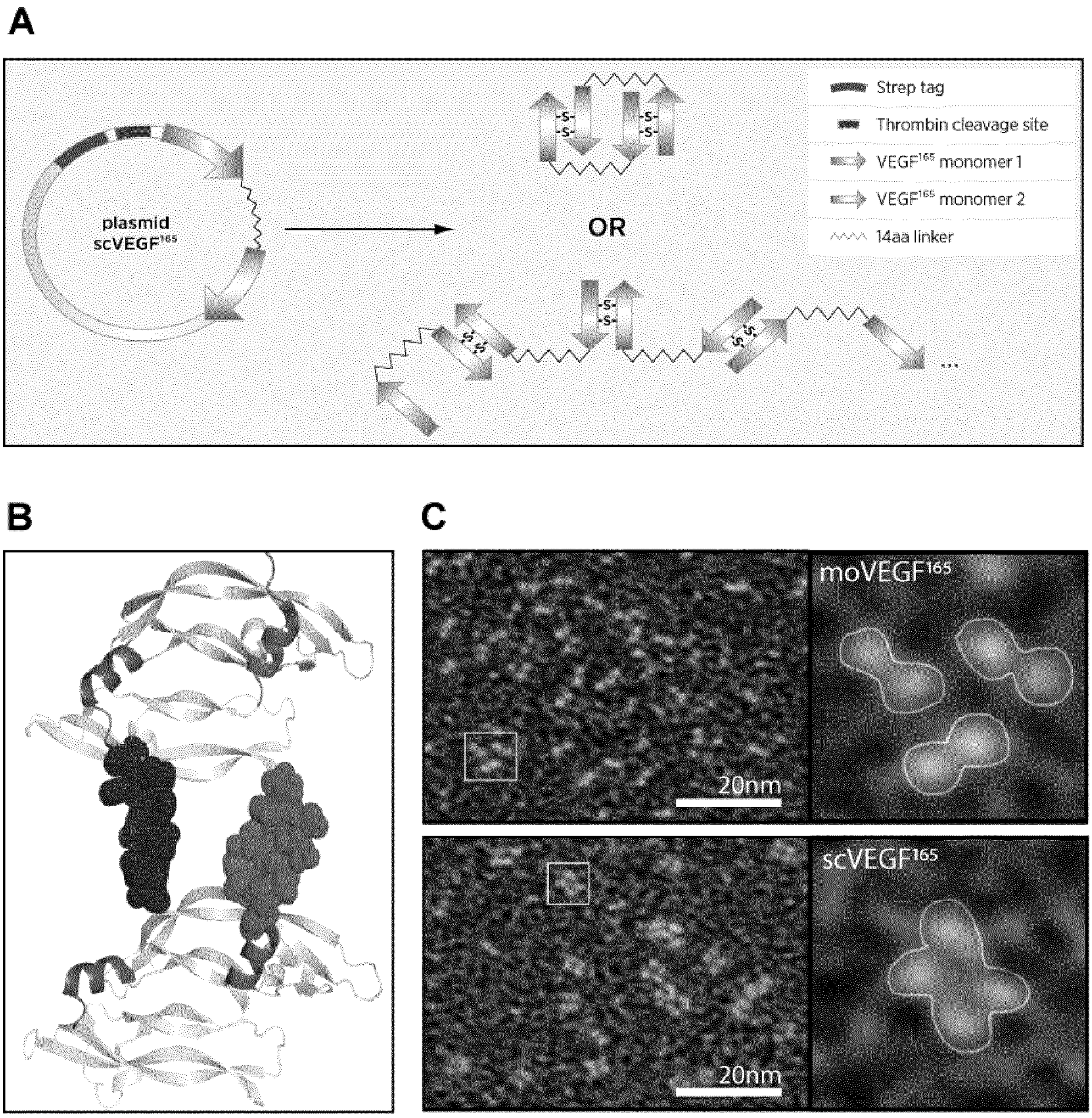
A
plasmid
scVEGF165
OR
Strep tag
Thrombin cleavage site
VEGF165 monomer 1
VEGF165 monomer 2
14aa linker
B
C
moVEGF165
scVEGF165
20nm
20nm

Figure 1

A

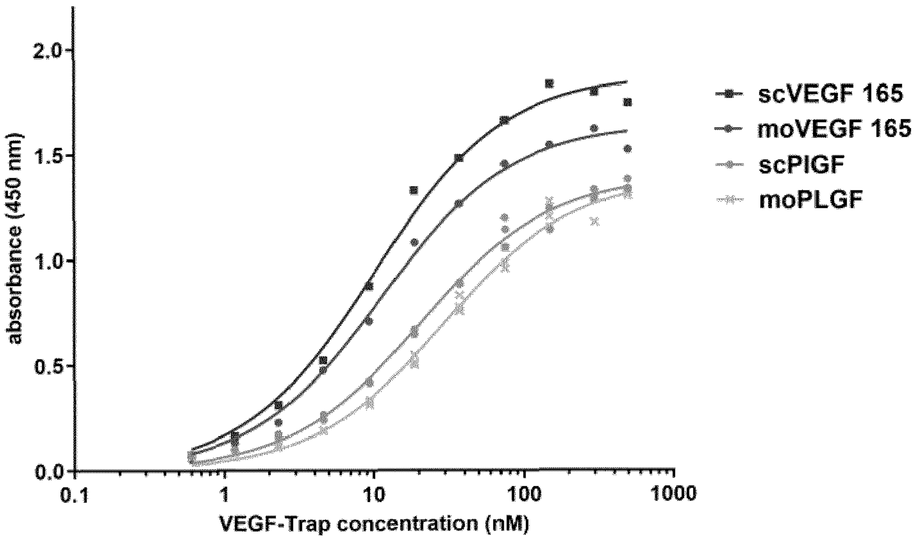
2.0
1.5
1.0
0.5
0.0
absorbance (450 nm)
0.1
1
10
100
1000
VEGF-Trap concentration (nM)
scVEGF 165
moVEGF 165
scPIGF
moPLGF

B

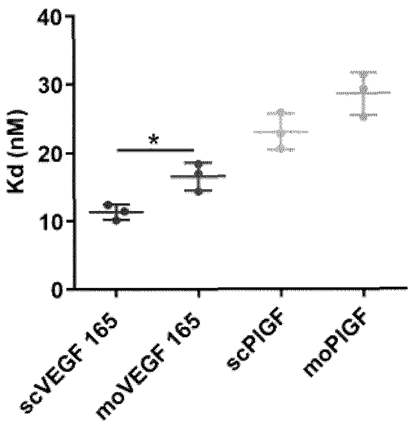
40
30
20
10
0
Kd (nM)
*
scVEGF 165
moVEGF 165
scPIGF
moPIGF

Figure 2

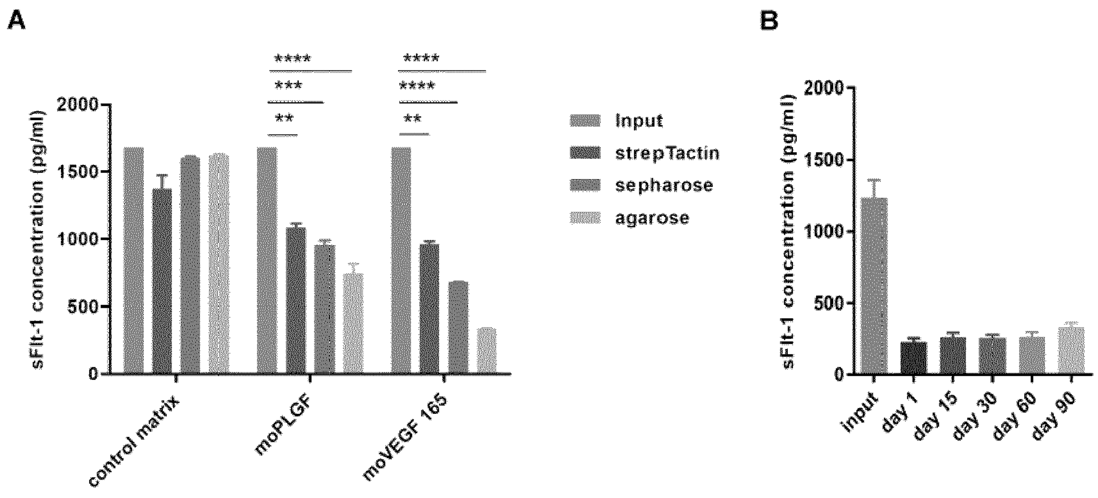
A
****
***
**
****
****
**
2000
1500
1000
500
0
sFlt-1 concentration (pg/ml)
control matrix
moPLGF
moVEGF 165
Input
strepTactin
sepharose
agarose
B
2000
1500
1000
500
0
sFlt-1 concentration (pg/ml)
input
day 1
day 15
day 30
day 60
day 90

Figure 3

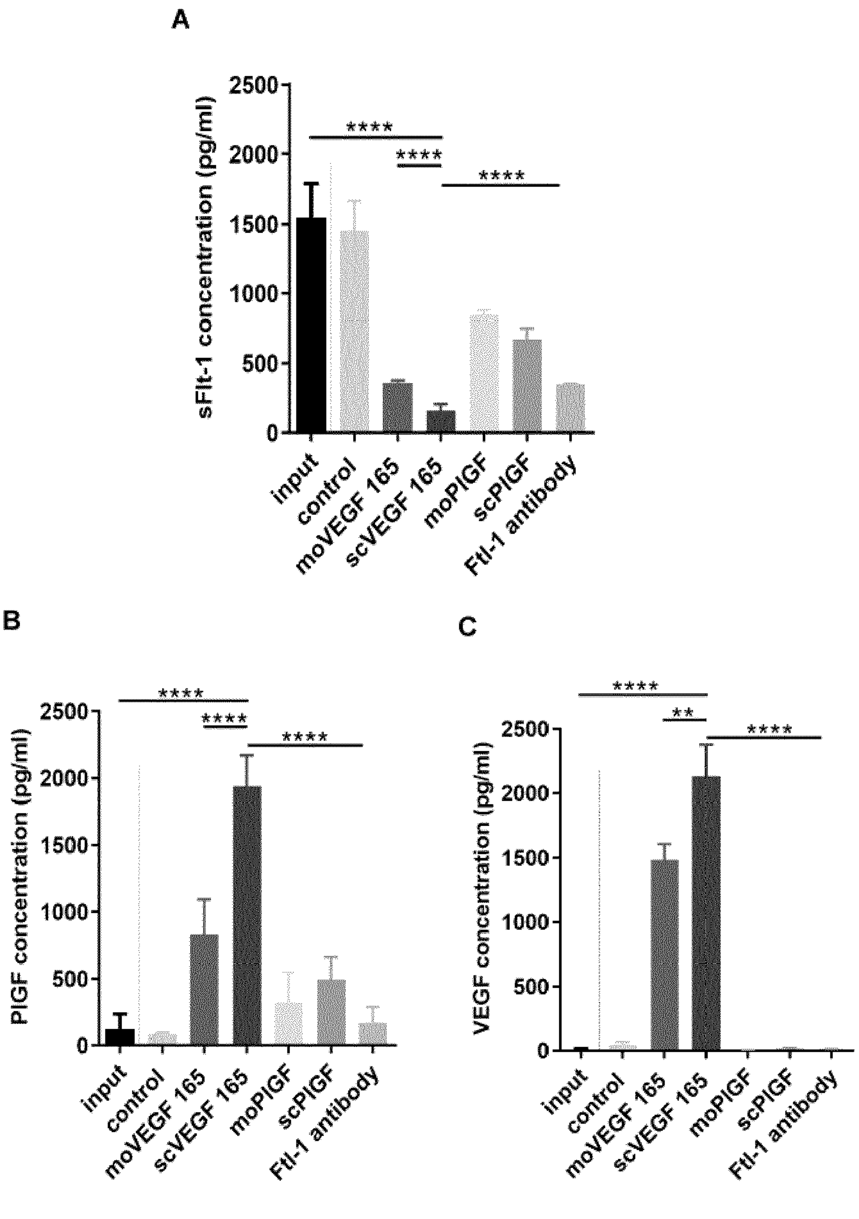
A
2500
2000
1500
1000
500
0
sFlt-1 concentration (pg/ml)
****
****
****
input
control
moVEGF 165
scVEGF 165
moPlGF
scPlGF
Ftl-1 antibody
B
2500
2000
1500
1000
500
0
PlGF concentration (pg/ml)
****
****
****
input
control
moVEGF 165
scVEGF 165
moPlGF
scPlGF
Ftl-1 antibody
C
2500
2000
1500
1000
500
0
VEGF concentration (pg/ml)
****
**
****
input
control
moVEGF 165
scVEGF 165
moPlGF
scPlGF
Ftl-1 antibody

Figure 4

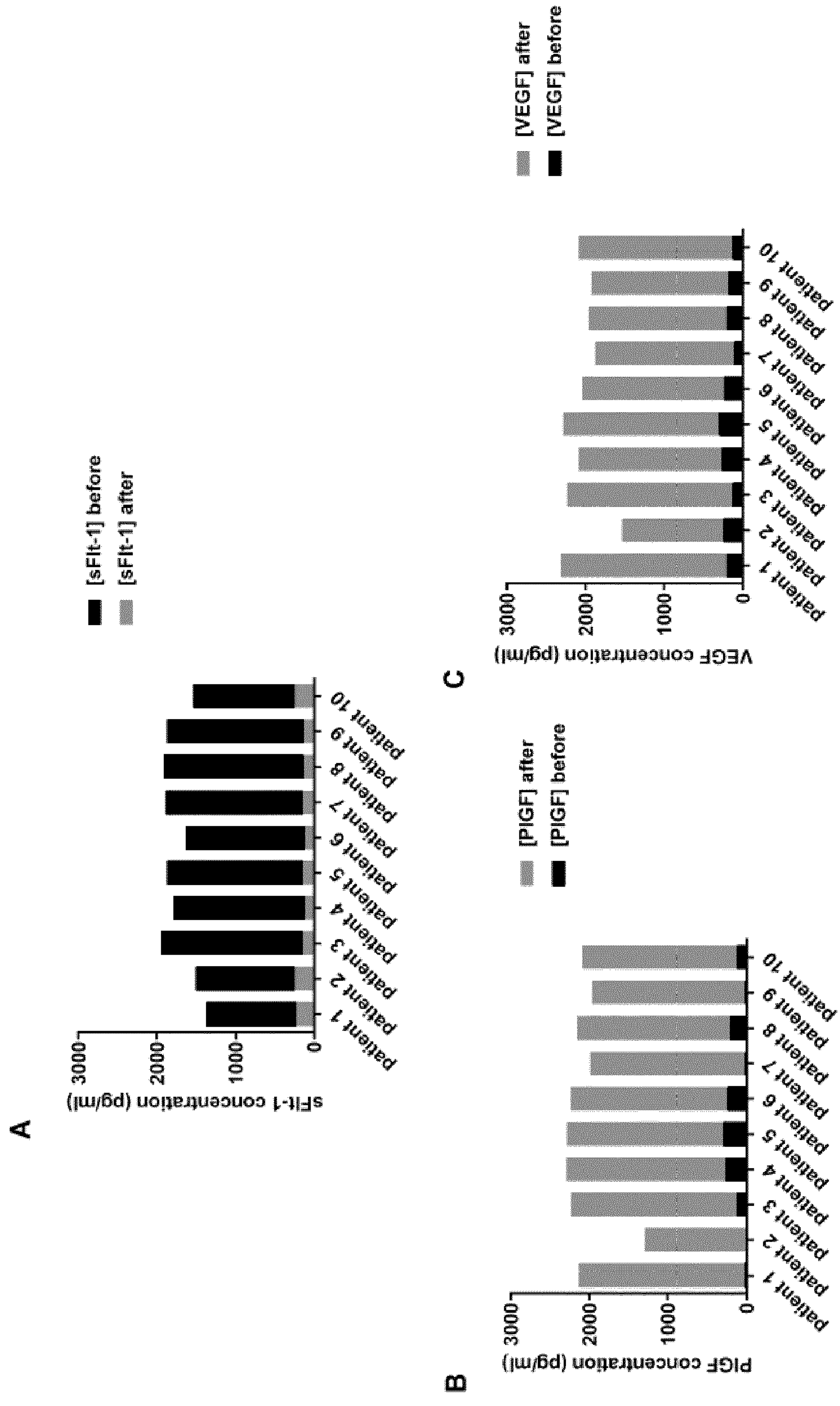
A
sFlt-1 concentration (pg/ml)
3000
2000
1000
0
patient 1
patient 2
patient 3
patient 4
patient 5
patient 6
patient 7
patient 8
patient 9
patient 10
[sFlt-1] before
[sFlt-1] after
B
PlGF concentration (pg/ml)
[PlGF] after
[PlGF] before
C
VEGF concentration (pg/ml)
[VEGF] after
[VEGF] before

Figure 5

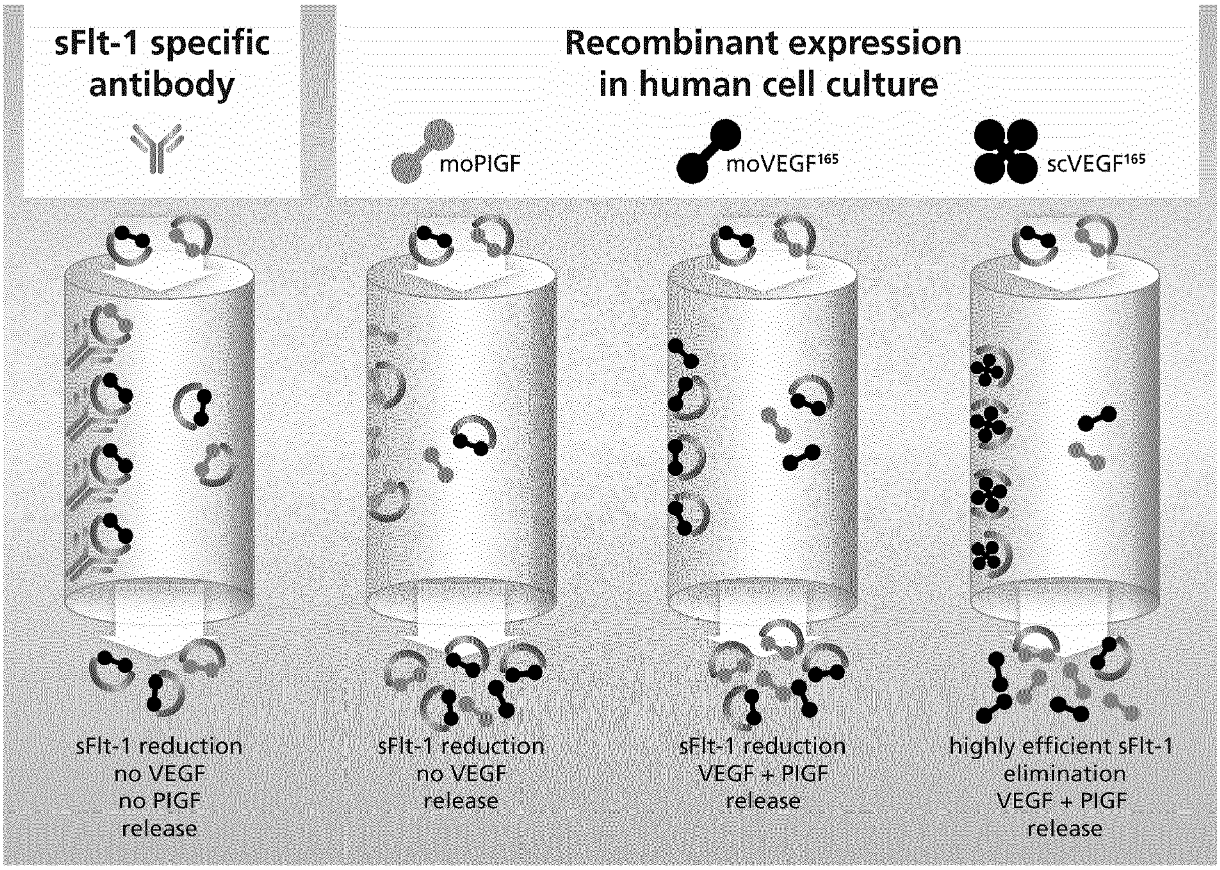
sFlt-1 specific antibody
Recombinant expression in human cell culture
moPlGF
moVEGF165
scVEGF165
sFlt-1 reduction no VEGF no PlGF release
sFlt-1 reduction no VEGF release
sFlt-1 reduction VEGF + PlGF release
highly efficient sFlt-1 elimination VEGF + PlGF release

**Figure 6**

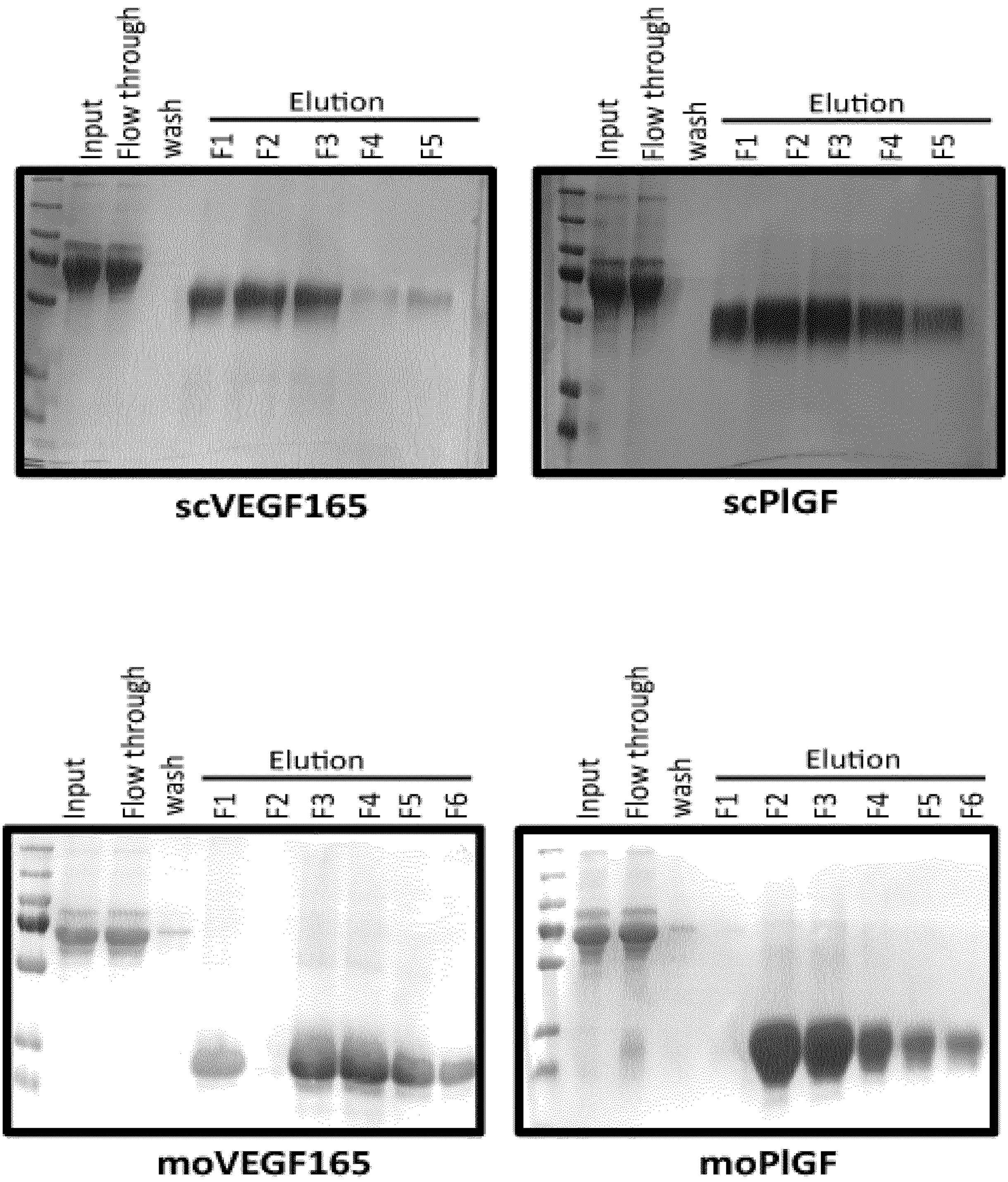
Input
Flow through
wash
Elution
F1
F2
F3
F4
F5
scVEGF165
Input
Flow through
wash
Elution
F1
F2
F3
F4
F5
scPlGF
Input
Flow through
wash
Elution
F1
F2
F3
F4
F5
F6
moVEGF165
Input
Flow through
wash
Elution
F1
F2
F3
F4
F5
F6
moPlGF

**Figure 7**

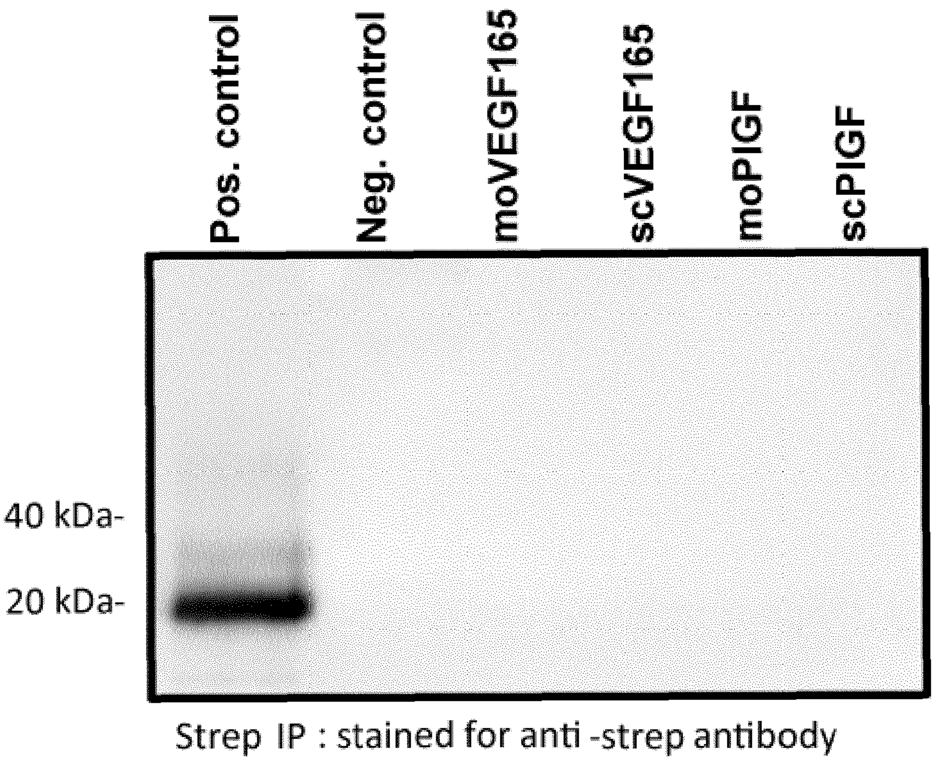
Pos. control
Neg. control
moVEGF165
scVEGF165
moPIGF
scPIGF
40 kDa-
20 kDa-
Strep IP : stained for anti -strep antibody

**Figure 8**

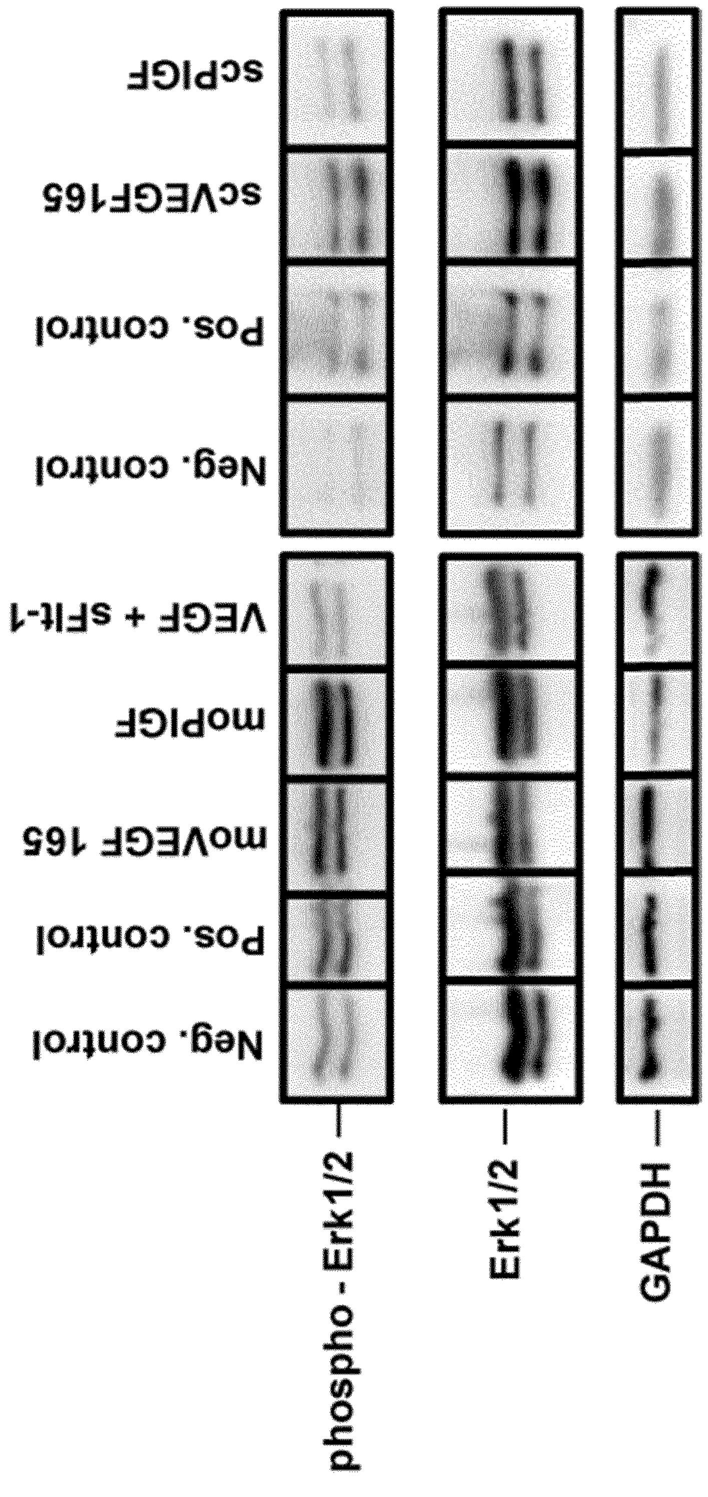
Neg. control
Pos. control
moVEGF 165
moPlGF
VEGF + sFlt-1
Neg. control
Pos. control
scVEGF165
scPlGF
phospho - Erk1/2
Erk1/2
GAPDH

Figure 9

Europäisches Patentamt
European Patent Office
Office européen des brevets

# EUROPEAN SEARCH REPORT

Application Number
EP 20 16 4619

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | MAHSA MATIN ET AL: "Affinity-Enhanced Multimeric VEGF (Vascular Endothelial Growth Factor) and PlGF (Placental Growth Factor) Variants for Specific Adsorption of sFlt-1 to Restore Angiogenic Balance in Preeclampsia", HYPERTENSION., 6 July 2020 (2020-07-06), XP055718134, US ISSN: 0194-911X, DOI: 10.1161/HYPERTENSIONAHA.120.14974 * the whole document * ----- | 1-17 | INV. C07K14/71 A61M1/34 A61P9/12 |
| X,D | US 2010/247650 A1 (SMITH HENRY J [US] ET AL) 30 September 2010 (2010-09-30) * paragraphs [0026] - [0034]; claims 1, 9 * ----- | 1-17 | |
| X | LAURA TRAPIELLA-ALFONSO ET AL: "VEGF (Vascular Endothelial Growth Factor) Functionalized Magnetic Beads in a Microfluidic Device to Improve the Angiogenic Balance in Preeclampsia", HYPERTENSION, vol. 74, no. 1, 1 July 2019 (2019-07-01), pages 145-153, XP055719177, US ISSN: 0194-911X, DOI: 10.1161/HYPERTENSIONAHA.118.12380 * abstract; figures 3,4 * ----- | 1-17 | TECHNICAL FIELDS SEARCHED (IPC) C07K A61M A61P |
| X | WO 2012/109282 A2 (AGAMIN PHARMACEUTICALS LLC [US]; KUSSIE PAUL [US]; JOO WOO S [US]) 16 August 2012 (2012-08-16) * paragraphs [0006], [0007]; example 1 * ----- -/-- | 1-17 | |

The present search report has been drawn up for all claims

1

| Place of search | Date of completion of the search | Examiner |
|---|---|---|
| Munich | 4 August 2020 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 16 4619

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SANDRO DE FALCO: "The discovery of placenta growth factor and its biological activity", EXPERIMENTAL AND MOLECULAR MEDICINE, vol. 44, no. 1, 1 January 2012 (2012-01-01), page 1, XP055719666, KR ISSN: 1226-3613, DOI: 10.3858/emm.2012.44.1.025 * page 1, paragraph 4; figure 1 * ----- | 2-4,6,7 | |
| X | NICHOLAS J GASPAR ET AL: "Cysteine 116 participates in intermolecular bonding of the human VEGF121 homodimer", ARCHIVES OF BIOCHEMISTRY AND BIOPHYSICS, ACADEMIC PRESS, US, vol. 404, no. 1, 1 August 2002 (2002-08-01), pages 126-135, XP008153488, ISSN: 0003-9861, DOI: 10.1016/S0003-9861(02)00239-4 * figure 1 * ----- | 2-4,6,7 | |
| | | | TECHNICAL FIELDS SEARCHED (IPC) |

The present search report has been drawn up for all claims

1

| Place of search | Date of completion of the search | Examiner |
|---|---|---|
| Munich | 4 August 2020 | Krüger, Julia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO. EP 20 16 4619

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2020

| Patent document cited in search report | Publication date | Patent family member(s) | Publication date |
|---|---|---|---|
| US 2010247650 A1 | 30-09-2010 | NONE | |
| WO 2012109282 A2 | 16-08-2012 | CA 2826563 A1 | 16-08-2012 |
| | | EP 2672996 A2 | 18-12-2013 |
| | | ES 2683953 T3 | 28-09-2018 |
| | | JP 6169495 B2 | 26-07-2017 |
| | | JP 2014511375 A | 15-05-2014 |
| | | US 2014065150 A1 | 06-03-2014 |
| | | WO 2012109282 A2 | 16-08-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20100247650 A1

### Non-patent literature cited in the description


- **SIBAI BM.** Prevention of preeclampsia: a big disappointment. *Am J Obstet Gynecol.,* 1998, vol. 179, 1275-1278
- **REDMAN CW.** Latest Advances in Understanding Preeclampsia. *Science,* 2005, vol. 308, 1592-1594
- **WALKER JJ.** Pre-eclampsia. *Lancet,* 2000, vol. 356, 1260-1265
- **MAYNARD SE ; MIN J-Y ; MERCHAN J ; LIM K-H ; LI J ; MONDAL S ; LIBERMANN TA ; MOR-GAN JP ; SELLKE FW ; STILLMAN IE.** Ex-cess placental soluble fms-like tyrosine kinase 1 (sFlt1) may contribute to endo-thelial dysfunction, hypertension, and proteinuria in preeclampsia. *J Clin Invest.,* 2003, vol. 111, 649-658
- **LEVINE RJ ; MAYNARD SE ; QIAN C ; LIM K-H ; ENGLAND LJ ; YU KF ; SCHISTERMAN EF ; THADHANI R ; SACHS BP ; EPSTEIN FH.** Circulating angiogenic factors and the risk of preeclampsia. *N Engl J Med.,* 2004, vol. 350, 672-683
- ACOG Practice Bulletin No. 202: Gestational Hypertension and Preeclampsia. *Obstetrics & Gynecology,* 2019, vol. 133, e1-e25
- **MAKRIS A ; YEUNG KR ; LIM SM ; SUNDERLAND N ; HEFFERNAN S ; THOMPSON JF ; ILI-OPOULOS J ; KILLINGSWORTH MC ; YONG J ; XU B.** Placental Growth Factor Reduces Blood Pressure in a Utero-placental Ischemia Model of Preeclampsia in Nonhuman Primates. *Hyperten-sion.,* 2016, vol. 67, 1263-1272
- **LI Z ; ZHANG Y ; YING MA J ; KAPOUN AM ; SHAO Q ; KERR I ; LAM A ; O'YOUNG G ; SANNAJUST F ; STATHIS P.** Re-combinant vascular endothelial growth factor 121 attenuates hypertension and improves kidney damage in a rat model of preeclampsia. *Hypertension,* 2007, vol. 50, 686-692
- **EDDY AC ; BIDWELL III GL ; GEORGE EM.** Pro-angiogenic therapeutics for preeclampsia. *Biol Sex Differ.,* 2018, vol. 9, 36
- **TURANOV AA ; LO A ; HASSLER MR ; MAKRIS A ; ASHAR-PATEL A ; ALTERMAN JF ; COLES AH ; HARASZTI RA ; ROUX L ; GODINHO BMDC.** RNAi modulation of placental sFLT1 for the treatment of preeclampsia. *Nature Biotechnology,* 2018, vol. 36, 1164-1173
- **FAN X ; RAI A ; KAMBHAM N ; SUNG JF ; SINGH N ; PETITT M ; DHAL S ; AGRAWAL R ; SUT-TON RE ; DRUZIN ML.** Endometrial VEGF induces placental sFLT1 and leads to pregnancy complications. *Journal of Clinical Investigation,* 2014, vol. 124, 4941-4952
- **THADHANI R ; KISNER T ; HAGMANN H ; BOSSUNG V ; NOACK S ; SCHAARSCHMIDT W ; JANK A ; KRIBS A ; CORNELY OA ; KREYSSIG C.** Pilot Study of Extracorporeal Removal of Soluble Fms-Like Tyrosine Kinase 1 in Preeclampsia. *Circulation,* 2011, vol. 124, 940-950
- **THADHANI R ; HAGMANN H ; SCHAARSCHMIDT W ; ROTH B ; CINGOEZ T ; KARUMANCHI SA ; WENGER J ; LUCCHESI KJ ; TAMEZ H ; LINDNER T.** Removal of Soluble Fms-Like Tyrosine Kinase-1 by Dextran Sulfate Apheresis in Preeclampsia. *J Am Soc Nephrol.,* 2016, vol. 27, 903-913
- **TRAPIELLA-ALFONSO L ; ALEXANDRE L ; FRAICHARD C ; PONS K ; DUMAS S ; HUART L ; GAUCHER JF ; HEBERT-SCHUSTER M ; GUIBOURDENCHE J ; FOURNIER T.** VEGF (Vascular Endothelial Growth Factor) Functionalized Magnetic Beads in a Microfluidic Device to Improve the Angiogenic Balance in Preeclampsia. *Hypertension,* July 2019, vol. 74 (1), 145-153
- **JAIN A ; CHENG K.** The principles and applications of avidin-based nanoparticles in drug delivery and diagnosis. *Journal of Controlled Release,* 2017, vol. 245, 27-40
- **CLAFFEY KP ; SENGER DR ; SPIEGELMAN BM.** Structural requirements for dimeriza-tion, glycosylation, secretion, and biological function of VPF/VEGF. *Biochimica et Biophysica Acta (BBA) - Protein Structure and Molecular Enzymology,* 1995, vol. 1246, 1-9

- **PERETZ D ; GITAY-GOREN H ; SAFRAN M ; KIMMEL N ; GOSPODAROWICZ D ; NEUFELD G.** Glycosylation of vascular endothelial growth factor is not required for its mito-genic activity. *Biochemical and Biophysical Research Communications,* 1992, vol. 182, 1340-1347
- **KURIAKOSE A ; CHIRMULE N ; NAIR P.** Immunogenicity of Biotherapeutics: Causes and Association with Posttranslational Modifications. *Journal of Immunology Research,* 2016, 1-18
- **TATUSOVA et al.** *FEMS Microbiol. Lett.,* 1999, vol. 174, 247-250
- **MIZUSHIMA ; NAGATA.** pEF-BOS, a powerful mammalian expression vector. *Nucleic Acids Res.,* 1990, vol. 18 (17), 5322
- **MULLIGAN RC ; HOWARD BH ; BERG P.** Synthesis of rabbit beta-globin in cultured monkey kidney cells following infection with a SV40 beta-globin recombinant genome. *Nature,* 1979, vol. 277 (5692), 108-14
- **AGARWAL P ; ZWOLANEK D ; KEENE DR ; SCHULZ J-N ; BLUMBACH K ; HEINEGÅRD D ; ZAUCKE F ; PAULSSON M ; KRIEG T ; KOCH M.** Collagen XII and XIV, New Partners of Cartilage Oligomeric Matrix Protein in the Skin Extracellular Matrix Suprastructure. *Journal of Biological Chemistry,* 2012, vol. 287, 22549-22559
- **BOBER M ; ENOCHSSON C ; COLLIN M ; MORGELIN M.** Collagen VI Is a Subepithelial Adhesive Target for Human Respiratory Tract Pathogens. *Journal of Innate Immunity,* 2010, vol. 2, 160-166
- **CHRISTINGER HW ; FUH G ; DE VOS AM ; WIESMANN C.** The Crystal Structure of Pla-cental Growth Factor in Complex with Domain 2 of Vascular Endothelial Growth Factor Receptor-1. *Journal of Biological Chemistry,* 2004, vol. 279, 10382-10388

SEQUENCE LISTING

<110> Universität zu Köln

<120> VEGF dimer molecules and columns comprising a VEGF dimer molecule as well as uses, production methods and methods involving the same

<130> P72781EP

<160> 13

<170> PatentIn version 3.5

<210> 1
<211> 232
<212> PRT
<213> Homo sapiens

<400> 1

```
Met Asn Phe Leu Leu Ser Trp Val His Trp Ser Leu Ala Leu Leu Leu
1               5                   10                  15


Tyr Leu His His Ala Lys Trp Ser Gln Ala Ala Pro Met Ala Glu Gly
            20                  25                  30


Gly Gly Gln Asn His His Glu Val Val Lys Phe Met Asp Val Tyr Gln
        35                  40                  45


Arg Ser Tyr Cys His Pro Ile Glu Thr Leu Val Asp Ile Phe Gln Glu
    50                  55                  60


Tyr Pro Asp Glu Ile Glu Tyr Ile Phe Lys Pro Ser Cys Val Pro Leu
65                  70                  75                  80


Met Arg Cys Gly Gly Cys Cys Asn Asp Glu Gly Leu Glu Cys Val Pro
                85                  90                  95


Thr Glu Glu Ser Asn Ile Thr Met Gln Ile Met Arg Ile Lys Pro His
            100                 105                 110


Gln Gly Gln His Ile Gly Glu Met Ser Phe Leu Gln His Asn Lys Cys
        115                 120                 125


Glu Cys Arg Pro Lys Lys Asp Arg Ala Arg Gln Glu Lys Lys Ser Val
    130                 135                 140


Arg Gly Lys Gly Lys Gly Gln Lys Arg Lys Arg Lys Lys Ser Arg Tyr
145                 150                 155                 160


Lys Ser Trp Ser Val Tyr Val Gly Ala Arg Cys Cys Leu Met Pro Trp
                165                 170                 175
```

Ser Leu Pro Gly Pro His Pro Cys Gly Pro Cys Ser Glu Arg Arg Lys
180 185 190

His Leu Phe Val Gln Asp Pro Gln Thr Cys Lys Cys Ser Cys Lys Asn
195 200 205

Thr Asp Ser Arg Cys Lys Ala Arg Gln Leu Glu Leu Asn Glu Arg Thr
210 215 220

Cys Arg Cys Asp Lys Pro Arg Arg
225 230

<210> 2
<211> 26
<212> PRT
<213> Homo sapiens

<400> 2

Met Asn Phe Leu Leu Ser Trp Val His Trp Ser Leu Ala Leu Leu Leu
1 5 10 15

Tyr Leu His His Ala Lys Trp Ser Gln Ala
20 25

<210> 3
<211> 167
<212> PRT
<213> Artificial Sequence

<220>
<223> Example of a first VEGF molecule

<400> 3

Ala Ser Ala Pro Met Ala Glu Gly Gly Gly Gln Asn His His Glu Val
1 5 10 15

Val Lys Phe Met Asp Val Tyr Gln Arg Ser Tyr Cys His Pro Ile Glu
20 25 30

Thr Leu Val Asp Ile Phe Gln Glu Tyr Pro Asp Glu Ile Glu Tyr Ile
35 40 45

Phe Lys Pro Ser Cys Val Pro Leu Met Arg Cys Gly Gly Cys Cys Asn
50 55 60

Asp Glu Gly Leu Glu Cys Val Pro Thr Glu Glu Ser Asn Ile Thr Met
65 70 75 80

Gln Ile Met Arg Ile Lys Pro His Gln Gly Gln His Ile Gly Glu Met

```
                85                  90                  95

Ser Phe Leu Gln His Asn Lys Cys Glu Cys Arg Pro Lys Lys Asp Arg
            100                 105                 110

Ala Arg Gln Glu Asn Pro Cys Gly Pro Cys Ser Glu Arg Arg Lys His
        115                 120                 125

Leu Phe Val Gln Asp Pro Gln Thr Cys Lys Cys Ser Cys Lys Asn Thr
    130                 135                 140

Asp Ser Arg Cys Lys Ala Arg Gln Leu Glu Leu Asn Glu Arg Thr Cys
145                 150                 155                 160

Arg Cys Asp Lys Pro Arg Arg
                165


<210>  4
<211>  170
<212>  PRT
<213>  Artificial Sequence

<220>
<223>  Example of a second VEGF molecule

<400>  4

Thr Arg Ala Pro Met Ala Glu Gly Gly Gly Gln Asn His His Glu Val
1               5                   10                  15

Val Lys Phe Met Asp Val Tyr Gln Arg Ser Tyr Cys His Pro Ile Glu
            20                  25                  30

Thr Leu Val Asp Ile Phe Gln Glu Tyr Pro Asp Glu Ile Glu Tyr Ile
        35                  40                  45

Phe Lys Pro Ser Cys Val Pro Leu Met Arg Cys Gly Gly Cys Cys Asn
    50                  55                  60

Asp Glu Gly Leu Glu Cys Val Pro Thr Glu Glu Ser Asn Ile Thr Met
65                  70                  75                  80

Gln Ile Met Arg Ile Lys Pro His Gln Gly Gln His Ile Gly Glu Met
                85                  90                  95

Ser Phe Leu Gln His Asn Lys Cys Glu Cys Arg Pro Lys Lys Asp Arg
            100                 105                 110

Ala Arg Gln Glu Asn Pro Cys Gly Pro Cys Ser Glu Arg Arg Lys His
        115                 120                 125
```

```
Leu Phe Val Gln Asp Pro Gln Thr Cys Lys Cys Ser Cys Lys Asn Thr
    130                 135                 140

Asp Ser Arg Cys Lys Ala Arg Gln Leu Glu Leu Asn Glu Arg Thr Cys
145                 150                 155                 160

Arg Cys Asp Lys Pro Arg Arg Gly Ser Thr
                165                 170


<210>  5
<211>  351
<212>  PRT
<213>  Artificial Sequence

<220>
<223>  Example of a VEGF dimer molecule

<400>  5

Ala Ser Ala Pro Met Ala Glu Gly Gly Gly Gln Asn His His Glu Val
1               5                   10                  15

Val Lys Phe Met Asp Val Tyr Gln Arg Ser Tyr Cys His Pro Ile Glu
            20                  25                  30

Thr Leu Val Asp Ile Phe Gln Glu Tyr Pro Asp Glu Ile Glu Tyr Ile
        35                  40                  45

Phe Lys Pro Ser Cys Val Pro Leu Met Arg Cys Gly Gly Cys Cys Asn
    50                  55                  60

Asp Glu Gly Leu Glu Cys Val Pro Thr Glu Glu Ser Asn Ile Thr Met
65                  70                  75                  80

Gln Ile Met Arg Ile Lys Pro His Gln Gly Gln His Ile Gly Glu Met
                85                  90                  95

Ser Phe Leu Gln His Asn Lys Cys Glu Cys Arg Pro Lys Lys Asp Arg
            100                 105                 110

Ala Arg Gln Glu Asn Pro Cys Gly Pro Cys Ser Glu Arg Arg Lys His
        115                 120                 125

Leu Phe Val Gln Asp Pro Gln Thr Cys Lys Cys Ser Cys Lys Asn Thr
    130                 135                 140

Asp Ser Arg Cys Lys Ala Arg Gln Leu Glu Leu Asn Glu Arg Thr Cys
145                 150                 155                 160
```

```
Arg Cys Asp Lys Pro Arg Arg Gly Ser Thr Ser Gly Ser Gly Lys Ser
                165                 170                 175

Ser Glu Gly Lys Gly Thr Arg Ala Pro Met Ala Glu Gly Gly Gly Gln
            180                 185                 190

Asn His His Glu Val Val Lys Phe Met Asp Val Tyr Gln Arg Ser Tyr
        195                 200                 205

Cys His Pro Ile Glu Thr Leu Val Asp Ile Phe Gln Glu Tyr Pro Asp
    210                 215                 220

Glu Ile Glu Tyr Ile Phe Lys Pro Ser Cys Val Pro Leu Met Arg Cys
225                 230                 235                 240

Gly Gly Cys Cys Asn Asp Glu Gly Leu Glu Cys Val Pro Thr Glu Glu
                245                 250                 255

Ser Asn Ile Thr Met Gln Ile Met Arg Ile Lys Pro His Gln Gly Gln
            260                 265                 270

His Ile Gly Glu Met Ser Phe Leu Gln His Asn Lys Cys Glu Cys Arg
        275                 280                 285

Pro Lys Lys Asp Arg Ala Arg Gln Glu Asn Pro Cys Gly Pro Cys Ser
    290                 295                 300

Glu Arg Arg Lys His Leu Phe Val Gln Asp Pro Gln Thr Cys Lys Cys
305                 310                 315                 320

Ser Cys Lys Asn Thr Asp Ser Arg Cys Lys Ala Arg Gln Leu Glu Leu
                325                 330                 335

Asn Glu Arg Thr Cys Arg Cys Asp Lys Pro Arg Arg Gly Ser Thr
            340                 345                 350


<210>  6
<211>  14
<212>  PRT
<213>  Artificial Sequence

<220>
<223>  Example of a linker

<400>  6

Gly Ser Thr Ser Gly Ser Gly Lys Ser Ser Glu Gly Lys Gly
1               5                   10
```

<210> 7
<211> 1053
<212> DNA
<213> Artificial Sequence

<220>
<223> scVEGF165 as used in the examples

<400> 7
gctagcgctc ctatggctga aggcggagga cagaatcacc acgaggtggt caagttcatg 60

gacgtgtacc agcggagcta ctgtcacccc atcgagacac tggtggacat cttccaagag 120

taccccgacg agatcgagta catcttcaag cccagctgcg tgcccctgat gagatgtggc 180

ggctgctgca atgacgaagg cctggaatgt gtgcccaccg aggaatccaa catcaccatg 240

cagatcatgc ggatcaagcc ccaccagggc cagcatatcg gcgagatgtc tttcctgcag 300

cacaacaagt gcgagtgcag acccaagaag gaccgggcca gacaagagaa tccttgcggc 360

ccttgcagcg agcggagaaa gcacctgttt gtgcaggacc ctcagacctg caagtgctcc 420

tgcaagaaca ccgacagccg gtgcaaagcc agacagctgg aactgaacga gcggacctgc 480

agatgcgaca agcctagaag aggcagcaca agcggcagcg gcaaaagctc tgaaggcaag 540

ggaacgcgtg ccccaatggc agaaggtggc ggccagaacc accatgaggt cgtgaagttt 600

atggatgtct atcagcggtc ctactgccat cctatcgaaa ccctggtcga tatttttcaa 660

gagtatccgg atgagattga gtatattttc aaaccctcct gtgtgccgct catgcgctgc 720

ggcggatgct gtaatgatga gggacttgag tgcgtgccaa ccgaagagtc taatattacg 780

atgcagatta tgagaatcaa accgcatcaa gggcagcata ttggggaaat gagcttcctc 840

cagcataaca aatgtgaatg ccggccgaag aaggacagag cccggcaaga aaacccatgc 900

ggcccctgtt ccgagaggcg gaaacatctg ttcgttcaag atccccagac ctgtaaatgt 960

agctgtaaaa acaccgactc caggtgcaag gcccggcaac tcgagctgaa cgagagaaca 1020

tgtcgctgcg ataagcccag acggggatcc aca 1053

<210> 8
<211> 846
<212> DNA
<213> Artificial Sequence

<220>
<223> scPIGF as used in the examples

<400> 8
acagctagcc tgcctgctgt tcctcctcaa caatgggccc tgtctgccgg caatggcagc 60

tctgaagttg aggtggtgcc cttccaagaa gtgtggggca gaagctactg cagagccctg 120

gaaagactgg tggacgtggt gtctgagtac cccagcgagg tggaacacat gttcagccct 180

agctgcgtgt ccctgctgag atgcacaggc tgttgcggcg acgagaatct gcactgcgtg 240

ccagtggaaa ccgccaacgt gacaatgcag ctgctgaaaa tcagaagcgg cgacagaccc 300

agctacgtgg aactgacctt cagccagcac gtccgctgcg agtgtagacc cctgcgggaa 360

aagatgaagc ccgagagatg cggagatgcc gtgcctagaa gaggcagcac atctggctct 420

ggcaagagca gcgaaggcaa gggacttcct gctgtgccac cacagcagtg ggcactgagt 480

gccggaaatg gctcctctga ggtggaagtc gtgccttttc aagaagtctg ggacgctcc 540

tactgtcgcg ctcttgagag actggtcgat gtcgtcagcg agtacccctc cgaagtcgag 600

cacatgtttt ccccatcctg tgtgtctctg ctgcggtgta ccggatgctg cggggatgag 660

aacctgcatt gtgtgcctgt cgagacagcc aatgtcacca tgcagctcct caagatcaga 720

tccggcgatc ggccctccta cgtcgagctg acattttctc agcacgttcg atgcgaatgc 780

cggcctctgc gcgagaaaat gaagcctgaa cgctgtggcg acgccgtgcc tcgtagagga 840

tccaca 846

<210> 9
<211> 282
<212> PRT
<213> Artificial Sequence

<220>
<223> Protein sequence of scPIGF as used in the examples

<400> 9

Thr Ala Ser Leu Pro Ala Val Pro Pro Gln Gln Trp Ala Leu Ser Ala
1 5 10 15

Gly Asn Gly Ser Ser Glu Val Glu Val Val Pro Phe Gln Glu Val Trp
20 25 30

Gly Arg Ser Tyr Cys Arg Ala Leu Glu Arg Leu Val Asp Val Val Ser
35 40 45

Glu Tyr Pro Ser Glu Val Glu His Met Phe Ser Pro Ser Cys Val Ser
50 55 60

Leu Leu Arg Cys Thr Gly Cys Cys Gly Asp Glu Asn Leu His Cys Val
65 70 75 80

Pro Val Glu Thr Ala Asn Val Thr Met Gln Leu Leu Lys Ile Arg Ser
85 90 95

Gly Asp Arg Pro Ser Tyr Val Glu Leu Thr Phe Ser Gln His Val Arg
100 105 110

Cys Glu Cys Arg Pro Leu Arg Glu Lys Met Lys Pro Glu Arg Cys Gly
115 120 125

```
Asp Ala Val Pro Arg Arg Gly Ser Thr Ser Gly Ser Gly Lys Ser Ser
    130                 135                 140

Glu Gly Lys Gly Leu Pro Ala Val Pro Pro Gln Gln Trp Ala Leu Ser
145                 150                 155                 160

Ala Gly Asn Gly Ser Ser Glu Val Glu Val Val Pro Phe Gln Glu Val
                165                 170                 175

Trp Gly Arg Ser Tyr Cys Arg Ala Leu Glu Arg Leu Val Asp Val Val
            180                 185                 190

Ser Glu Tyr Pro Ser Glu Val Glu His Met Phe Ser Pro Ser Cys Val
        195                 200                 205

Ser Leu Leu Arg Cys Thr Gly Cys Cys Gly Asp Glu Asn Leu His Cys
    210                 215                 220

Val Pro Val Glu Thr Ala Asn Val Thr Met Gln Leu Leu Lys Ile Arg
225                 230                 235                 240

Ser Gly Asp Arg Pro Ser Tyr Val Glu Leu Thr Phe Ser Gln His Val
                245                 250                 255

Arg Cys Glu Cys Arg Pro Leu Arg Glu Lys Met Lys Pro Glu Arg Cys
            260                 265                 270

Gly Asp Ala Val Pro Arg Arg Gly Ser Thr
        275                 280


<210>  10
<211>  29
<212>  DNA
<213>  Artificial Sequence

<220>
<223>  Primer scVEGF165 forward

<400>  10
acagctagcg ctcctatggc tgaaggcgg                                          29


<210>  11
<211>  29
<212>  DNA
<213>  Artificial Sequence

<220>
<223>  Primer scVEGF165 reverse

<400>  11
```

```
tgtggatccc cgtctgggct tatcgcagc                                    29


<210>  12
<211>  28
<212>  DNA
<213>  Artificial Sequence

<220>
<223>  Primer scPlGF forward

<400>  12
acagctagcc tgcctgctgt tcctcctc                                     28


<210>  13
<211>  29
<212>  DNA
<213>  Artificial Sequence

<220>
<223>  Primer scPlGF reverse

<400>  13
tgtggatccc tctacgaggc acggcgtcg                                    29
```